# EUROPÄISCHE PATENTSCHRIFT

(11) **EP 1 395 781 B1**
(45) Veröffentlichungstag und Bekanntmachung des Hinweises auf die Patenterteilung: **09.08.2006**
(21) Anmeldenummer: 02747345.3
(22) Anmeldetag: 05.06.2002
(51) Int. Cl.: F24F 6/08, F24F 6/02, A61L 9/14

(54) **VORRICHTUNG ZUR ANREICHERUNG VON LUFT MIT EINEM LUFTBEHANDLUNGSMITTEL INSBESONDERE ZUR LUFTENTKEIMUNG, LUFTBEDUFTUNG UND/ODER GERUCHSMASKIERUNG**
DEVICE FOR ENRICHING AIR WITH AN AIR TREATMENT AGENT, ESPECIALLY FOR SANITIZING AND/OR SCENTING AIR AND/OR MASKING ODORS
DISPOSITIF POUR TRAITER L'AIR AVEC UN AGENT DE TRAITEMENT DE L'AIR, NOTAMMENT POUR ASEPTISER, PARFUMER L'AIR ET/OU MASQUER DES ODEURS

(30) Priorität: 13.06.2001 DE 10128563; 25.08.2001 DE 10141734
(43) Veröffentlichungstag der Anmeldung: 10.03.2004
(73) Patentinhaber: Schuer, Joerg Peter, 41844 Wegberg-Dalheim (DE)
(72) Erfinder: Schuer, Joerg Peter, 41844 Wegberg-Dalheim (DE)
(74) Vertreter: von Kirschbaum, Alexander
(86) Internationale Anmeldenummer: PCT/EP2002/006157
(87) Internationale Veröffentlichungsnummer: WO 2002/101299

(56) Entgegenhaltungen:
- EP-A- 0 713 060
- WO-A-01/03747
- US-A- 2 419 477
- US-A- 5 664 730
- PATENT ABSTRACTS OF JAPAN vol. 002, no. 135 (M-039), 10. November 1978 (1978-11-10) & JP 53 105051 A (TOSHIBA ELECTRIC APPLIANCE CO LTD), 12. September 1978 (1978-09-12)

## Beschreibung

Die Erfindung betrifft eine Vorrichtung zur Anreicherung von Luft mit einem Luftbehandlungsmittel insbesondere zur Luftentkeimung, Luftbeduftung und Geruchsmaskierung.

Die Luftbehandlung ist beispielsweise in Wohnräumen (z. B. von Allergikern), in Bürogebäuden, Verkehrs- und Transportmitteln, Hygienebereichen und im Gesundheitswesen erforderlich. Zur Luftbehandlung sind Verdampfer bekannt, in denen mit Hilfe einer Verdampfungseinrichtung ein Luftbehandlungsmittel verdampft wird. Bei einer derartigen Verdampfung des Luftbehandlungsmittels wird die Luft relativ stark mit Behandlungsmitteln angereichert, so dass sich das Behandlungsmittel in dem zu behandelnden Raum niederschlägt. Auch durch eine Taktung eines aufgrund von Wärmezufuhr arbeitenden Verdampfers kann ein Niederschlag des Luftbehandlungsmittels nicht vermieden werden. Der Niederschlag ist lediglich zeitlich begrenzt. Der Niederschlag von Luftbehandlungsmittel an kalten Gegenständen wie Fenstern u. dgl. stört den Benutzer und führt ferner zur leichteren Verschmutzung dieser Gegenstände, da beispielsweise Staub durch den Niederschlag angezogen wird. Der Niederschlag an Holzmöbeln u. dgl. kann ferner zu Beschädigungen an den Möbeln führen.

Ferner sind Sprüh-Druckluft-Systeme zum Versprühen von Luftbehandlungsmittel bekannt. Hierbei erfolgt eine feine Zerstäubung des Luftbehandlungsmittels. Auch beim Verwenden von Sprüh-Druckluft-Systemen ist das Auftreten von Niederschlag des Luftbehandlungsmittels nicht vermieden.

Ein weiterer Anwendungsbereich für Luftbehandlungsmittel ist beispielsweise das Einbringen von Entkeimungsmitteln beim Abkühlen von Backwaren nach dem Backvorgang. Hierbei muss vermieden werden, dass sich vor dem Verpacken S.chimmelkeime auf der Oberfläche der Backwaren ablagern. Da auch in diesem Bereich ein Niederschlag des Luftbehandlungsmittels nicht akzeptabel ist, werden aufwendige Luftfilteranlagen mit unterschiedlichen Filtersystem eingesetzt. Hierbei besteht das Problem, dass sich die Schimmelkeime in der Luftfilteranlage ablagern können und für den Luftfilter selbst als Schimmelbildungsherde wirken. Dies hat zur Folge, dass die Filter häufig ausgewechselt und sehr gründlich gereinigt werden müssen.

Eine Luftbehandlung ist ferner bei der Lagerung von Käse nach der Reifung erforderlich, da nach der Reifung durch die in der Luft befindlichen Schimmelkeime eine unerwünschte Schimmelbildung auf der Käseoberfläche auftritt. Um dies zu vermeiden, werden Käse beispielsweise mit einem Deckmittel überzogen, in dem ein Antibiotikum enthalten ist. Das Antibiotikum dringt aufgrund von Diffusionen in den Außenbereich des Käses ein. Dies hat zur Folge, dass beim Genuss des Käses dem menschlichen Körper ungewünscht Antibiotikum zugeführt wird. Die Verwendung von Filteranlagen bei der Käsezubereitung hat denselben Nachteil wie bei der Zubereitung von Backwaren.

Aus EP 0 713 060 ist ein Luftbefeuchter bekannt. Der Luftbefeuchter weist ein oberes und ein unteres Reservoir auf, wobei aus einem oberen Reservoir Wasser durch Düsen bzw. Öffnungen in das untere Reservoir tropft. Das untere Reservoir weist einen Überlauf auf. Durch das überlaufende Wasser werden Textilbänder befeuchtet. Die Textilbänder sind mit Heizdrähten zur Verdampfung des Wassers umgeben.

Aufgabe der Erfindung ist es, eine Vorrichtung zur Anreicherung von Luft mit einem Luftbehandlungsmittel zu schaffen, bei der der Niederschlag des Luftbehandlungsmittels vermieden ist.

Die Lösung der Aufgabe erfolgt erfindungsgemäß durch die Merkmale des Anspruchs 1 bzw. 5.

Bei einer ersten bevorzugten Ausführungsform weist die erfindungsgemäße Vorrichtung einen Vorratsbehälter zur Aufnahme von flüssigem Luftbehandlungsmittel und eine mit dem Vorratsbehälter beispielsweise über ein Rohr oder einen Schlauch verbundene Verdampfungseinrichtung zum Erwärmen des Luftbehandlungsmittels auf. Erfindungsgemäß ist zwischen dem Vorratsbehälter und der Verdampfungseinrichtung, d. h. beispielsweise in dem Schlauch oder Rohr, eine Dosiereinrichtung angeordnet. Durch die Dosiereinrichtung wird eine mengenbegrenzte Zufuhr von flüssigem Luftbehandlungsmittel zu der Verdampfungseinrichtung gewährleistet. Erfindungsgemäß ist mit der Verdampfungseinrichtung ein Mischbehälter verbunden, in dem das verdampfte Luftbehandlungsmittel mit Luft gemischt wird. Zum Abführen des Gemisches aus Luft und dampfförmigen Luftbehandlungsmittel weist der Mischbehälter eine Austrittsöffnung auf. Erfindungsgemäß wird dem Mischbehälter beispielsweise durch Eintrittsöffnungen im Verhältnis zu der zugeführten geringen Menge an flüssigem Luftbehandlungsmittel derart viel Luft zugeführt, dass es möglich ist, dem zu behandelnden Raum einen Luftbehandlungsmittelanteil pro Stunde und Kubikmeter Luft zuzuführen, der zwischen 0,1 und 0,00001 ml vorzugsweise zwischen 0,01 und 0,001 ml beträgt. Aufgrund dieser geringen Menge an Luftbehandlungsmittel, die dem zu behandelnden Raum zugeführt wird, kann kein Niederschlag des Luftbehandlungsmittels in dem Raum nachgewiesen werden. Ein störender Niederschlag an kühlen Fenstern o. dgl. tritt daher nicht auf. Die erfindungsgemäße Vorrichtung ist somit insbesondere für Wohnräume, Wartezimmer u. dgl. geeignet..

In dem aus der Austrittsöffnung des Mischbehälters austretendem Gemisch aus dampfförmigen Luftbehandlungsmittel und Luft ist der Luftbehandlungsmittelanteil vorzugsweise kleiner als 100 ppb (parts per billion) und insbesondere kleiner als 10 ppb.

Vorzugsweise ist zum Mischen des dampfförmigen Luftbehandlungsmittels mit Luft dem Mischbehälter ein Mittel zur Erzeugung eines Luftstroms wie ein Ventilator zugeordnet. Von dem Ventilator wird Luft durch die im Mischbehälter vorgesehenen Lufteinlassöffnungen in den Mischbehälter eingesaugt oder eingeblasen. Ferner dient das Mittel zur Erzeugung eines Luftstroms dazu, das Gemisch aus Luft und dampfförmigem Luftbehandlungsmittel aus der Austrittsöffnung auszustoßen bzw. auszublasen.

Vorzugsweise wird eine Menge von 0,01 ml pro Kubikmeter und Stunde bis 0,005 ml pro Kubikmeter und Stunde an Luftbehandlungsmittel dem zu behandelnden Raum zugeführt. Bei einem Raum von beispielsweise 50 Kubikmeter Luft werden somit 0,5 ml pro Stunde bis 0,25 ml pro Stunde aus dem Vorratsbehälter der Verdampfungseinrichtung zugeführt und in dieser verdampft.

Insbesondere um ein Mitreißen von Tröpfchen an Luftbehandlungsmittel durch den Luftstrom zu vermeiden, darf die Förderleistung des Mittels zur Förderung des Luftstroms nicht zu groß sein. Andererseits ist ein relativ hohes Fördervolumen gefordert, um einen äußerst geringen Anteil an Luftbehandlungsmittel pro Kubikmeter Luft zu erzielen. Bei einer zu verdampfenden Menge an Luftbehandlungsmitteln von 0,25 - 0,5 ml pro Stunde beträgt der geförderte Volumenstrom an Luft vorzugsweise 25 - 35 m³ pro Stunde. Das Verhältnis zwischen geförderter Luftmenge und der Verdampfungseinrichtung zugeführten Menge Luftbehandlungsmittel liegt somit im Bereich von 140/1 bis 50/1, vorzugsweise 100/1 bis 70/1.

Um einen Unterdruck in dem Mischbehälter zu vermeiden, ist die mindestens eine Lufteintrittsöffnung des Mischbehälters im Querschnitt größer als die mindestens eine Austrittsöffnung. Bei mehreren Austritts- bzw. mehreren Eintrittsöffnungen ist jeweils die Summe der Querschnittsflächen der Austrittsöffnungen kleiner als die Summe der Querschnittsflächen der Lufteintrittsöffnungen.

Bei einer bevorzugten Ausführungsform der Erfindung erfolgt eine kontinuierliche Zufuhr von Luftbehandlungsmittel zu der Verdampfungseinrichtung. Um dies zu gewährleisten, weist die Dosiereinrichtung vorzugsweise eine Austrittsöffnung, durch die das Luftbehandlungsmittel in Richtung der Verdampfungseinrichtung austritt, auf, deren Querschnittsfläche kleiner als 0,078 cm, insbesondere kleiner als 0,000314 cm ist. Bei einer runden Austrittsöffnung entspricht dies einem Durchmesser von 0,1 mm bzw. 0,2 mm. Vorzugsweise handelt es sich um eine fest vorgegebene nicht variierbare Austrittsöffnung. Ein Variieren des Durchmessens der Austrittsöffnung kann beispielsweise durch Auswechseln entsprechender Scheibe o. dgl., in der die Austrittsöffnung vorgesehen ist, erfolgen. Ebenso ist es möglich, eine Scheibe o. dgl. mit relativ großer Austrittsöffnung vorzusehen und die Menge an Luftbehandlungsmittel, die der Verdampfungseinrichtung zugeführt wird, dadurch zu reduzieren, dass Scheiben mit kleineren Austrittsöffnungen in Flussrichtung vor oder hinter der Scheibe mit großen Luftaustrittsöffnungen vorgesehen werden. Hierfür können beispielsweise in einem zwischen dem Vorratsbehälter und der Verdampfungseinrichtung angeordneten Rohr oder Schlauch Einschiebschlitze zum Einschieben derartiger Scheiben vorgesehen sein.

Vorzugsweise ist der Vorratsbehälter gegenüber der Verdampfungseinrichtung derart angeordnet, dass ein Höhenunterschied zwischen diesen beiden Teilen der Vorrichtung besteht, wobei der Vorratsbehälter höher angeordnet ist. Hierdurch besteht ein Gefälle in der Fluidverbindung, wie dem Schlauch oder dem Rohr, zwischen dem Vorratsbehälter und der Verdampfungseinrichtung. Dies ermöglicht eine kontinuierliche Zufuhr von Luftbehandlungsmittel zu der Verdampfungseinrichtung, ohne dass eine Pumpe oder eine andere Fördereinrichtung vorgesehen sein muss. Die einzigen Energieverbraucher der erfindungsgemäßen Vorrichtung sind somit die Verdampfungseinrichtung und der Ventilator.

Da nur sehr geringe Mengen an Luftbehandlungsmittel verdampft werden müssen und es sich bei Luftbehandlungsmittel im Allgemeinen um relativ leicht flüchtige Stoffe handelt, weist die Verdampfungseinrichtung vorzugsweise eine Temperatur von 40 - 70 °C auf.

Es ist ferner möglich, anstatt einer Dosiereinrichtung mit entsprechend angepasster kleiner Austrittsöffnung eine Schlauchpumpe oder ein anderes geeignetes Fördermittel zum Fördern des Luftbehandlungsmittels vorzusehen. Hierbei erfolgt vorzugsweise wiederum eine kontinuierliche Förderung von Luftbehandlungsmittel. Die Menge des Luftbehandlungsmittels ist einerseits von dem Fördervolumen des Ventilators und andererseits von der Größe des zu behandelnden Raums abhängig. Anstatt einer kontinuierlichen Förderung ist es auch möglich, das Luftbehandlungsmittel in Intervallen der Verdampfungseinrichtung zuzuführen. Dies hat den Vorteil, dass herkömmliche kostengünstige Schlauchpumpen eingesetzt werden können.

Vorzugsweise weist der Vorratsbehälter eine Druckausgleichseinrichtung auf, so dass im Vorratsbehälter stets Umgebungsdruck und kein Unterdruck herrscht. Durch Unterdruck würde die Menge an Luftbehandlungsmittel, die der Verdampfungseinrichtung zugeführt wird, beeinflusst. Der Vorratsbehälter ist vorzugsweise mit einem Deckel o. dgl. verschlossen. Der Deckel, der zum Befüllen des Vorratsbehälters abnehmbar ist, weist vorzugsweise einen Partikelfilter auf. Der Partikelfilter ist derart ausgebildet, dass Luft in den Vorratsbehälter zum Druckausgleich einströmen kann, andererseits aber verhindert ist, dass Partikel wie beispielsweise Staub in den Vorratsbehälter gelangen. Durch derartige Partikel kann die Dosiereinrichtung verstopft oder die Durchflussrate beeinträchtigt werden. Die Druckausgleichseinrichtung, bei der es sich vorzugsweise um eine mit einem Partikelfilter versehene Öffnung handelt, ist vorzugsweise im Deckel vorgesehen. Sie kann jedoch auch an einer anderen Stelle des Vorratsbehälters angeordnet sein.

Eine zweite bevorzugte Ausführungsform der Erfindung zur Anreicherung von Luft mit einem Luftbehandlungsmittel weist ebenfalls einen Vorratsbehälter zur Aufnahme von flüssigem Luftbehandlungsmittel sowie eine mit dem Vorratsbehälter verbundene Verdampfungseinrichtung zum Erwärmen des Luftbehandlungsmittels auf. Diese bevorzugte Ausführungsform der Erfindung weist eine geneigt angeordnete Verdunstungsoberfläche der Verdunstungseinrichtung auf. Über diese geneigte Verdunstungsoberfläche fließt das Luftbehandlungsmittel. Erfindungsgemäß ist mit der Verdunstungseinrichtung eine Rückführeinrichtung verbunden, die das nicht verdampfte Luftbehandlungsmittel in den Vorratsbehälter zurückführt. Auf der Verdunstungsoberfläche der Verdunstungseinrichtung bildet sich somit vorzugsweise ein dünner Film an Luftbehandlungsmittel, so dass eine gleichmäßige Verdunstung des Luftbehandlungsmittels über die gesamte Oberfläche der Verdunstungsoberfläche stattfindet.

Durch das Vorsehen einer Rückführeinrichtung, durch die überschüssiges Luftbehandlungsmittel aufgefangen und in den Vorratsbehälter zurückgeführt wird, muss die Menge an Luftbehandlungsmittel, die der Verdunstungseinrichtung zugeführt wird, nicht so exakt bestimmt werden. Vielmehr ist die Menge an Luftbehandlungsmittel, die verdunstet und der zu behandelnden Luft zugeführt wird, im Wesentlichen von der Temperatur der Verdampfungsoberfläche der Verdampfungseinrichtung abhängig. Die Menge an Luftbehandlungsmittel, die der zu behandelnden Luft zugeführt wird, kann somit auf einfache Weise durch Regeln der Temperatur der Verdampfungsoberfläche eingestellt werden. Ferner weist die erfindungsgemäße Vorrichtung den Vorteil auf, dass aufgrund eines vorzugsweise kontinuierlichen Fließens des Luftbehandlungsmittels über die Verdampfungsoberfläche auf der Verdampfungsoberfläche keine Ablagerungen entstehen. Durch Ablagerungen kann der Wirkungsgrad der Verdampfungseinrichtung beeinträchtigt werden. Ferner ist es erforderlich, die Verdampfungseinrichtung regelmäßig zu reinigen. Derartiges Reinigen ist bei der erfindungsgemäßen Vorrichtung nicht oder allenfalls nur in sehr großen Zeitabständen erforderlich.

Die Neigung der Verdampfungsoberfläche, die vorzugsweise in einer Ebene angeordnet ist, beträgt vorzugsweise 10° bis 30° gegenüber einer Horizontalen. Besonders bevorzugt ist eine Neigung von 15° bis 25°.

Um ein gezieltes Fließen des Luftbehandlungsmittels über die Verdampfungsoberfläche zu gewährleisten, weist die Verdampfungsoberfläche vorzugsweise in Flussrichtung des Luftbehandlungsmittels verlaufende Transportrillen auf.

Vorzugsweise weist die zweite Ausführungsform der Erfindung einen entsprechend der vorstehend beschriebenen ersten Ausführungsform der Erfindung ausgebildeten Mischbehälter auf. Vorzugsweise ist innerhalb des Mischbehälters ein Mittel zur Erzeugung eines Luftstroms, wie ein Ventilator, angeordnet. Besonders bevorzugt ist die Anordnung des Ventilators unterhalb der Verdampfungseinrichtung, so dass die Luft seitlich an der Verdampfungseinrichtung vorbeiströmt und hierbei mit verdampften Luftverdampfungsmittel angereichert wird. Die Anordnung des Ventilators unterhalb der Verdampfungseinrichtung hat den Vorteil, dass die Verdampfungsoberfläche durch den vom Ventilator erzeugten Luftstrom nicht zu stark abgekühlt wird, so dass die gewünschte Verdampfungsrate erhalten bleibt.

Die zweite Ausführungsform der Erfindung mit geneigter Verdampfungsoberfläche kann entsprechend der vorstehend beschriebenen ersten Ausführungsform vorteilhaft weitergebildet sein.

Zum Einsatz der erfindungsgemäßen Vorrichtung zur Luftentkeimung wird als Luftbehandlungsmittel vorzugsweise eine antimikrobielle Zusammensetzung (X) verwendet. Vorzugsweise enthält die antimikrobielle Zusammensetzung eine, zwei oder mehrere GRAS(Generally Recognized As Safe)-Aromastoffe oder deren Derivate. Bevorzugte antimikrobielle Zusammensetzungen mit zwei oder mehreren GRAS-Aromastoffen sind in WO 01/03747 auf den Seiten 5 - 14 beschrieben auf die hiermit explizit Bezug genommen wird. Daneben können auch antimikrobielle Zusammensetzungen mit nur einem wie unten genauer definierten GRAS-Aromastoff eingesetzt werden, so z. B. Zusammensetzungen, die nur einen GRAS-Alkohol wie Propylenglykol oder Benzylalkohol enthalten.

Zur Geruchsneutralisierung der Luft wird vorzugsweise eine geruchsmaskierte Zusammensetzung (Y) verwendet, die wenigstens eine geruchsmaskierende Komponente (A) aufweist, die aus Terpenen, Maisstärke, Mangansalzen, ätherischen Ölen und Polyvinylpyrrolidon ausgewählt ist (DE 101 00 595).

Die bevorzugte Verbindung der geruchsmaskierenden Komponente (A) ist Polyvinylpyrrolidon (Polyvidone; Poly(2-oxo-1-Pyrrolidinyl)ethylen; Poly(1-vinyl-2-Pyrrolidon; nachfolgend auch kurz "PVP"), insbesondere solches PVP mit einer Molmasse von 10.000 bis 60.000 g/mol, vorzugsweise 30.000 bis 50.000 g/mol. Besonders bevorzugt ist PVP mit einer Molmasse von etwa 40.000 g/mol, d.h. es ist ein PVP, das einen gewissen Vernetzungsgrad (d. h. eine Viskosität von 15 bis 25, vorzugsweise etwa 2 mPa•s (20 Gew.-% in Wasser) besitzt. Der Anteil der geruchsmaskierenden Komponente (A) an der geruchsmaskierenden Zusammensetzung liegt vorzugsweise im Bereich von 0,001 bis 50 Gew.-%, besonders bevorzugt 0,1 bis 10 Gew.-%.

Gemäß der vorliegenden Erfindung kann die geruchsmaskierende Zusammensetzung (Y) eine weitere Functional-Flavour-Komponente (B) enthalten. Diese enthält vorzugsweise eine oder mehrere der folgenden Substanzen:
Hexylbutyrat, Octylacetat, Isobutylisobutyrat, cis-3-Hexen-1-ylacetat cis-3, γ-Decalactone, Ethylcaproat, Butylacetat, Ethylbenzoat, Ethylbutyrat, Hexylacetat, Methylcaproat, Phenylethylalkohol, Citronellol, Undecylaldehyd, Benzylphenylacetat, Cinnamik-Alkohol, Eugenol, Benzylacetat, Linalool, cis-Jasmone, Acetylmethylanthranilat, cis-3-Hexen-1-ol, cis-3-Hexen-1-ylsalicylat, Methylbenzoat, Methylsalicylat, Geranylacetat, cis-3-Hexen-1-ylacetat, Litsea Cubeba, Orangenöl, Phenylpropylalkohol und Phenylethylacetat.

Vorzugsweise beträgt der Anteil der Functional-Flavour-Komponente (B) 0,001 bis 20 Gew.-%, vorzugsweise 0,1 bis 5 Gew.-%, der geruchsmaskierenden Zusammensetzung.

Gemäß der vorliegenden Erfindung kann die geruchsmaskierende Zusammensetzung (Y) noch weiterhin eine Aromastoffkomponente (C) enthalten, die ausgewählt ist aus ätherischen Ölen, Aromastoffen und Duftstoffen. Dabei ist der Anteil der Aromastoffkomponente (C) an der geruchsmaskierenden Zusammensetzung 0,01 bis 95 Gew.-%, vorzugsweise 0,1 bis 80 Gew.-%.

In einer besonders bevorzugten Ausführungsform enthält die Aromastoffkomponente (C) antimikrobielle Substanzen, vorzugsweise enthält sie wenigstens einen GRAS(Generally Recognized As Safe)-Aromastoff. Besonders bevorzugt hiervon sind solche Aromastoffkomponenten (C) und auch solche vorstehend erwähnten antimikrobiellen Zusammensetzungen (X), die einen aromatischen GRAS-Aroma-Alkohol (wie Benzyalkohol, Zimtalkohol, α-Methylbenzylalkohol und Anisalkohol, wobei Benzylalkohol bevorzugt ist), einen lipophilen GRAS-Aroma-Alkohol wie Propylenglykol oder eine GRAS-Polyphenolverbindung enthalten, bzw. solche, die wenigstens 2 GRAS-Aromastoffe enthalten. Es hat sich dabei gezeigt, dass besonders solche Aromastoffkomponenten (C) bzw. antimikrobielle Zusammensetzungen (X), die
(a) einen oder mehrere GRAS-Aroma-Alkohole oder deren Derivate und
(b) einen oder mehrere Aromastoffe, ausgewählt aus
   (b1) Polyphenolverbindungen und
   (b2) GRAS-Aromasäuren oder deren Derivate
besonders geeignet sind.

Die genannten GRAS-Aroma-Alkohole der Komponente (a) sowie die nachfolgend definierten Komponenten (b) bis (h) sind von der FDA-Behörde zur Verwendung in Nahrungsmitteln als gewerbesicher anerkannt (GRAS = Generally Recognized As Safe In Food). Bei den erwähnten GRAS-Aroma-Alkoholen und auch bei den nachfolgend definierten anderen GRAS-Aromastoffen handelt es sich um solche Verbindungen, die in FEMA/FDA GRAS Flavour Substances Lists GRAS 3-15 Nr. 2001-3905 (Stand 2000) genannt sind. In dieser Liste sind natürliche und naturidentische Aromastoffe aufgeführt, die von der amerikanischen Gesundheitsbehörde FDA zur Verwendung in Nahrungsmitteln zugelassen sind: FDA Regulation 21 CFR 172.515 für naturidentische Aromastoffe (Synthetic Flavoring Substances and Adjuvants) und FDA Regulation 21 CFR 182.20 für natürliche Aromastoffe (Natural Flavoring Substances and Adjuvants).

Die Aromastoffkomponente (C) bzw. antimikrobielle Zusammensetzung (X) kann
0,1 bis 99,9 Gew.-%, vorzugsweise 0,5 bis 99 Gew.-%, Komponente (a),
0 bis 25 Gew.-%, vorzugsweise 0,01 bis 10 Gew.-%, Komponente (b1)
und/oder
0 bis 70 Gew.-%, vorzugsweise 0,01 bis 30 Gew.-%, Komponente (b2)
enthalten.

Erfindungsgemäß kann die Komponente (a) einen oder mehrere GRAS-Aroma-Alkoholen enthalten. Bevorzugt wird erfindungsgemäß der Einsatz von zwei oder drei GRAS-Aroma-Alkoholen. Im einzelnen können beispielsweise folgende GRAS-Aroma-Alkohole zum Einsatz kommen:
Benzylalkohol, Acetoin (Acetylmethylcarbinol), Ethylalkohol (Ethanol), Propylalkohol (1-Propanol), iso-Propylalkohol (2-Propanol, Isopropanol), Propylenglykol, Glycerin, n-Butylalkohol (n-Propylcarbinol), iso-Butylalkohol (2-Methyl-1-propanol), Hexylalkohol (Hexanol), L-Menthol, Octylalkohol (n-Octanol), Zimtalkohol (3-Phenyl-2-propen-1-ol), α-Methylbenzylalkohol (1-Phenylethanol), Heptylalkohol (Heptanol), n-Amylalkohol (1-Pentanol), isoAmylalkohol (3-Methyl-1-butanol), Anisalkohol (4-Methoxybenylalkohol, p-Anisalkohol), Citronellol, n-Decylalkohol (n-Decanol), Geraniol, β-γ-Hexanol (3-Hexenol), Laurylalkohol (Dodecanol), Linalool, Nerolidol, Nonadienol (2,6-Nonadien-1-ol), Nonylalkohol (Nonanol-1), Rhodinol, Terpineol, Borneol, Clineol (Eucalyptol), Anisol, Cuminylalkohol (Cuminol), 10-Undecen-1-ol, 1-Hexadecanol. Als Derivate können sowohl natürliche oder naturidentische Derivate als auch synthetische Derivate eingesetzt werden. Geeignete Derivate sind z. B. die Ester, Ether und Carbonate der vorstehend genannten GRAS-Aroma-Alkohole. Besonders bevorzugte GRAS-Aroma-Alkohole sind Benzylalkohol, 1-Propanol, Glycerin, Propylenglycol, n-Butylalkohol, Citronellol, Hexanol, Linalool, Acetoin und deren Derivate.

### Als Komponente (b1) können die folgenden Polyphenole eingesetzt werden:

Brenzcatechin, Resorcin, Hydrochinon, Phloroglucin, Pyrogallol, Cyclohexan, Usninsäure, Acylpolyphenole, Lignine, Anthocyane, Flavone, Catechine, Gallussäurederivate (z. B. Tannine, Gallotannin, Gerbsäuren, Gallus-Gerbsäuren), (einschließlich der Derivate der vorstehend genannten Verbindungen wie (2,5-Dihydroxyphenyl)carboxyl- und (2,5-Dihydroxyphenyl)alkylencarboxylsubstitutionen, Salze, Ester, Amide), Kaffesäure und deren Ester und Amide, Flavonoide (z. B. Flavon, Flavonol, Isoflavon, Gossypetin, Myrecetin, Robinetin, Apigenin, Morin, Taxifolin, Eriodictyol, Naringin, Rutin, Hesperidin, Troxerutin, Chrysin, Tangeritin, Luteolin; Catechine, Quercetin, Fisetin, Kaempferol, Galangin, Rotenoide, Aurone, Flavonole, -diole), Extrakte aus z. B. Camellia Primula. Weiterhin können auch deren mögliche Derivate, z. B. Salze, Säuren, Ester, Oxide und Ether verwendet werden. Das besonders bevorzugte Polyphenol ist Tannin (eine GRAS-Verbindung).

### Als Komponente (b2) können beispielsweise folgende GRAS-Säuren zum Einsatz kommen:

Essigsäure, Aconitsäure, Adipinsäure, Ameisensäure, Apfelsäure (1-Hydroxybernsteinsäure), Capronsäure, Hydrozimtsäure (3-Phenyl-1-propionsäure), Pelargonsäure (Nonansäure), Milchsäure (2-Hydroxypropionsäure), Phenoxyessigsäure (Glykolsäurephenylether), Phenylessigsäure (α-Toluolsäure), Valeriansäure (Pentansäure), isoValeriansäure (3-Methylbutansäure), Zimtsäure (3-Phenylpropensäure), Citronensäure, Mandelsäure (Hydroxyphenylessigsäure), Weinsäure (2,3-Dihydroxybutandisäure; 2,3-Dihydroxybernsteinsäure), Fumarsäure, Tanninsäure und deren Derivate.

Geeignete Derivate der genannten Säuren im Sinne der vorliegenden Erfindung sind Ester (z. B. C₁₋₆-Alkylester und Benzylester), Amide (einschließlich N-substituierte Amide) und Salze (Alkali-, Erdalkali- und Ammoniumsalze). Ebenfalls umfaßt der Begriff Derivate im Sinne der vorliegenden Erfindung Modifikationen der Seitenketten-Hydroxyfunktionen (z. B. Acyl- und Alkylderivate) und Modifikationen der Doppelbindungen (z. B. die perhydrierten und hydroxilierten Derivate der genannten Säuren).

Das Mischungsverhältnis der Komponente (a) zu Komponenten (b) liegt vorzugsweise zwischen 10.000 : 1 und 1 : 10.000, besonders bevorzugt zwischen 1000 : 1 und 1:1000 und ganz besonders bevorzugt zwischen 100 : 1 und 1 : 100.

In einer bevorzugten Ausführungsform der erfindungsgemäßen Vorrichtung enthält die Aromastoffkomponente (C') bzw. die antimikrobielle Zusammensetzung (X)
(a1) Benzylalkohol als notwendigen Bestandteil und gegebenenfalls
(a2) einen oder mehrere weitere GRAS-Aroma-Alkohole oder deren Derivate und
(b1) eine oder mehrere Polyphenolverbindungen und/oder
(b2) eine oder mehrere GRAS-Säuren oder deren Derivate.

Geeignete Mengen der Komponenten (a1), (a2), (b1) und (b2) sind dabei:
0,1 bis 99 Gew.-%, vorzugsweise 0,1 bis 75 Gew.-% Benzylakohol;
0 bis 99,8 Gew.-%, vorzugsweise 0,01 bis 99 Gew.-% Komponente (a2);
0 bis 25 Gew.-%, vorzugsweise 0,01 bis 10 Gew.-% Komponente (b1)
   und/oder
0 bis 70 Gew.-%, vorzugsweise 0,01 bis 30 Gew.-% Komponente (b2).

Die Aromastoffkomponente (C') bzw. die antimikrobielle Zusammensetzung (X) kann weiterhin noch die folgenden Komponenten (c) bis (h) enthalten, die ebenfalls Aromastoffe sind, die in der FEMA/FDA GRAS Flavour Substances Liste als G.R.A.S. (Generally Recognized As Safe In Food) 3-15 Nr. 2001-3905 (Stand 2000) anerkannt sind.

Als Komponente (c) können folgende Phenolverbindungen zum Einsatz kommen:
Thymol, Methyleugenol, Acetyleugenol, Safrol, Eugenol, Isoeugenol, Anethol, Phenol, Methylchavicol (Estragol; 3-4-Methoxyphenyl-1-propen), Carvacrol, α-Bisabolol, Fornesol, Anisol (Methoxybenzol) und Propenylguaethol (5-Prophenyl-2-ethoxaphenol) und deren Derivate.

Als GRAS-Ester (Komponente (d)) kommen Allicin und die folgenden Acetate iso-Amylacetat (3-Methyl-1-butylacetat), Benzylacetat, Benzylphenylacetat, n-Butylacetat, Cinnamylacetat (3-Phenylpropenylacetat), Citronellylacetat, Ethylacetat (Essigester), Eugenolacetat (Acetyleugenol), Geranylacetat, Hexylacetat (Hexanylethanoat), Hydrocinnamylacetat (3-Phenyl-propylacetet), Linalylacetat, Octylacetat, Phenylethylacetat, Terpinylacetat, Triacetin (Glyceryltriacetat), Kaliumacetat, Natriumacetat, Calciumacetat zum Einsatz. Weitere geeignete Ester sind die Esterderivate der vorstehend definierten Säuren (Komponente (b2)).

Als Terpene (Komponente (e)) kommen z. B. Campher, Limonen und β-Caryophyllen in Betracht.

Zu den verwendbaren Acetalen (Komponente (f)) zählen z. B. Acetal, Acetaldehyddibutylacetal, Acetaldehyddipropylacetal, Acetaldehydphenethylpropylacetal, Zimtaldehydethylenglycolacetal, Decanaldimethylacetal, Heptanaldimethylacetal, Heptanalglycerylacetal und Benzaldehydpropylenglykolacetal.

Als Aldehyde (Komponente (g)) sind z. B. Acetylaldehyd, Anisaldehyd, Benzaldehyd, iso-Butylaldehyd (Methyl-1-propanal), Citral, Citronellal, n-Caprinaldehyd (n-Decanal), Ethylvanillin, Fufurol, Heliotropin (Piperonal), Heptylaldehyd (Heptanal), Hexylaldehyd (Hexanal), 2-Hexenal (β-Propylacrolein), Hydrozimtaldehyd (3-Phenyl-1-propanal), Laurylaldehyd (Docdecanal), Nonylaldehyd (n-Nonanal), Octylaldehyd (n-Octanal), Phenylacetaldehyd (1-Oxo-2-phenylethan), Propionaldehyd (Propanal), Vanillin, Zimtaldehyd (3-Phenylpropenal), Perillaaldehyd und Cuminaldehyd verwendbar.

Erfindungsgemäß einsetzbar sind beispielsweise auch die im folgenden aufgeführten etherischen Öle und/oder die alkoholischen, glykolischen oder durch CO₂-Hochdruckverfahren erhaltenen Extrakte aus den genannten Pflanzen (Komponente (h)):
(h1) Öle bzw. Extrakte mit hohem Anteil an Alkoholen: Melisse, Koriander, Kardamon, Eukalyptus;
(h2) Öle bzw. Extrakte mit hohem Anteil an Aldehyden: Eukalyptus citriodora, Zimt, Zitrone, Lemongras, Melisse, Citronella, Limette, Orange;
(h3) Öle bzw. Extrakte mit hohem Anteil an Phenolen: Oreganum, Thymian, Rosmarin, Orange, Nelke, Fenchel, Campher, Mandarine, Anis, Cascarille, Estragon und Piment;
(h4) Öle bzw. Extrakte mit hohem Anteil an Acetaten: Lavendel;
(h5) Öle bzw. Extrakte mit hohem Anteil an Estern: Senf, Zwiebel, Knoblauch;
(h6) Öle bzw. Extrakte mit hohem Anteil an Terpenen: Pfeffer, Pomeranze, Kümmel, Dill, Zitrone, Pfefferminz, Muskatnuss.

Der Anteil der Komponenten (c) - (h) in der Aromastoffkomponente (C) bzw. (C') und in der antimikrobiellen Zusammensetzung (X) ist vorzugsweise kleiner oder gleich 25 Gew.-% und liegt bevorzugt im Bereich von 0,001 bis 9 Gew.-%. Bevorzugt unter den weiteren GRAS-Aromastoffen sind die Phenole (c) und etherischen Öle (h).

Besonders bevorzugt im Sinne der vorliegenden Erfindung sind Aromstoffkomponente (C) bzw. (C') und antimikrobielle Zusammensetzung (X), deren antimikrobiell wirksamer Bestandteil ausschließlich aus GRAS-Aromastoffen besteht, d. h. keine "Derivate" der GRAS-Aromastoffe enthält. Als Beispiel einer solchen Zusammensetzung ist ein Gemisch aus Benzylalkohol, einem oder zwei der vorstehend genannten GRAS-Aroma-Alkohole (a2) und Tannin zu nennen. Dieses Gemisch enthält dabei vorzugsweise 0,1 - 99,9, besonders bevorzugt 0,1 - 20 Gew.-% Benzylalkohol und 0,01 - 10 Gew.-% Tannin. Ein weiteres Beispiel einer bevorzugten Zusammensetzung ist ein Gemisch aus 2 Alkoholen, einem Polyphenol (insbesondere Tannin) und einem etherischen Öl (insbesondere einem phenolischen etherischen Öl, Komponente (h3)).

Neben den Komponenten (A) bis (C) können in den Zusammensetzungen (X) und (Y) zusätzlich noch weitere Verbindungen (D) wie Alkohole (D1) Emulgatoren (D2), Stabilisatoren (D3), Antioxidantien (D4), Konservierungsmittel (D5), Lösemittel (D6), Trägerstoffe (D7) etc. eingesetzt werden. Der Anteil der Komponenten (D) an der geruchsmaskierenden Zusammensetzung darf bis 99 Gew.-% betragen, ist vorzugsweise kleiner als 50 Gew.-% und liegt besonders bevorzugt im Bereich von 0,1 bis 20 Gew.-%.

Bei den Alkoholen (D1) handelt es sich erfindungsgemäß um einwertige oder mehrwertige Alkohole mit 2 bis 10 C-Atomen, vorzugsweise mit 2 bis 7 C-Atomen, wobei die GRAS-Alkohole (a) hiervon nicht umfaßt sind. Vorzugsweise werden solche Mengen an GRAS-Aroma-Alkoholen (a) und weiteren Alkoholen (D1) eingesetzt, daß deren Mischungsverhältnis zwischen 1000 : 1 und 1 : 1000, insbesondere zwischen 100 : 1 und 1 : 100 und besonders bevorzugt zwischen 10 : 1 und 1 : 10 liegt.

Bei den Trägerstoffen D7 handelt es sich vorzugsweise um polymere Verbindungen wie Polyethylenglykol, Polypropylenglykol usw.

Es kann bei bestimmten Anwendungen, z.B. wenn die geruchsmaskierende Zusammensetzung mit Lebensmittel in Kontakt kommt oder in von Personen bewohnten Räumen eingesetzt wird, angebracht sein, dass Systeme eingesetzt werden, die frei von Ethanol und Isopropanol bzw. frei von bedenklichen Dosierungen von Ethanol und Isopropanol sind, da diese Stoffe z. B. von Lebensmitteln absorbiert werden können und auch von den Personen in den behandelten Räumen eingeatmet werden können. Darüber hinaus kann bei der Verwendung dieser Verbindungen Explosionsgefahr bestehen.

Insbesondere kann die vorstehend beschriebene Vorrichtung auch in Verbindungen mit einer Kühleinrichtung wie einem Haushaltskühlschrank verwendet werden. Es ist hierdurch möglich, eine geringe Menge an Luftbehandlungsmittel, insbesondere Luftentkeimungsmittel oder geruchsmaskierendes Luftbehandlungsmittel, in den Innenraum des Kühlschranks bzw. der Kühleinrichtung einzubringen.

Eine weitere bevorzugte Verwendung der erfindungsgemäßen Vorrichtung besteht in Verbindung mit einem Computer. Durch die in einem Computer auftretenden Wärme und des sich in der Luft ansammelnden Staubs wird von dem Lüfter eine große Anzahl an Keimen in die Raumluft eingebracht. Vorzugsweise wird daher im Bereich des Gebläses eines Computers die erfindungsgemäße Vorrichtung angeordnet. Nun kann der von dem Computergebläse erzeugte Luftstrom zum Abführen des Luftbehandlungsmittels genutzt werden. Somit wird die mit Keimen versehene Abluft des Computers sofort durch ein Luftbehandlungsmittel behandelt. Ferner kann die erfindungsgemäße Vorrichtung im Luftansaugbereich des Lüfters angeordnet werden, so dass bereits die Keimbildung unterdrückt wird.

Sowohl bei der Kühleinrichtung als auch bei dem Computer ist es möglich, die erfindungsgemäße Vorrichtung außen am Gehäuse oder innerhalb der Kühleinrichtung bzw. des Computers vorzusehen. Es ist ferner möglich die Wärmequellen des Kühlschranks oder des Computers als Verdampfungseinrichtung zu nutzen. Insbesondere beim Computer kann ggf. eine zusätzliche Verdampfungsanrichtung vollständig entfallen.

Nachfolgend wird die Erfindung anhand bevorzugter Ausführungsformen unter Bezugnahme auf die anliegenden Zeichnungen näher erläutert.

Es zeigen:
- Fig. 1: einen schematische Seitenansicht einer bevorzugten Ausführungsform der Erfindung,
- Fig. 2: eine vergrößerte Ansicht des Bereichs A in Fig. 1,
- Fig. 3: eine schematische Seitenansicht einer weiteren bevorzugten Ausführungsform der Erfindung,
- Fig. 4: eine schematische Seitenansicht einer weiteren bevorzugten Ausführungsform der Erfindung,
- Fig. 5: eine Schnittansicht entlang der Linie V-V in Fig. 4,
- Fig. 6: eine schematische Schnittansicht entlang der Linie VI-VI in Fig. 4,
- Fig.7: eine schematische Ansicht eines Haushaltskühlschranks mit einer außerhalb des Kühlschranks angebrachten Vorrichtung zur Luftanreicherung,
- Fig. 8: eine schematische Darstellung eines Haushaltskühlschranks mit innerhalb des Kühlschranks angeordneter Vorrichtung für Luftanreicherung und
- Fig. 9: eine schematische Ansicht eines Computers mit erfindungsgemäßer Vorrichtung zur Luftanreicherung.

Die erfindungsgemäße Vorrichtung weist einen Vorratsbehälter 10 zur Aufnahme von Luftbehandlungsmittel 12 auf. Der Behälter 10 ist im dargestellten Ausführungsbeispiel zylindrisch. Es kann sich jedoch auch um einen rechteckigen oder anders geformten Vorratsbehälter handeln. Ein Boden 14 des Vorratsbehälters 10 ist in Richtung einer Auslassöffnung 16 geneigt, so dass das Luftbehandlungsmittel 12 in Richtung der Auslassöffnung 16 fließt, auch wenn nur noch eine geringe Menge an Luftbehandlungsmittel 12 in dem Vorratsbehälter 10 enthalten ist. Das Volumen des Vorratsbehälters 10 ist derart bemessen, dass der Vorratsbehälter 10 mit einer Menge an Luftbehandlungsmittel 12, die für etwa einen Monat ausreichend ist, gefüllt werden kann.

Zum Befüllen des Vorratsbehälters 10 weist dieser eine Einfüllöffnung 18 auf, die mit einem zylindrischen Einfüllstützen 20 verbunden ist. Der Einfüllstützen 20 ist mit einem im dargestellten Ausführungsbeispiel kegelstumpfförmigen Deckel 22 verschlossen. Zur Ausbildung von Lufteintrittsöffnungen 24, die auch bei geschlossenem Deckel 22 offen bleiben, ist das in Richtung des Deckels 22 weisende Ende des zylindrischen Ansatzes 22 mit Zinnen o. dgl. versehen. Aufgrund der Lufteintrittsöffnungen 24 ist gewährleistet, dass in dem Vorratsbehälter 10 stets Umgebungsdruck herrscht und keine Beeinflussung der geförderten Menge an Luftbehandlungsmittel aufgrund von Unterdruck auftreten kann.

An der Auslassöffnung 16 ist ein Auslassstutzen 26 angeordnet, an dem über einen Flansch 28 ein Rohr 30 befestigt ist. Um zu verhindern, dass Staub oder andere Partikel in das Rohr 30 gelangen, kann beispielsweise in der Auslassöffnung 16 ein feinmaschiger Filter vorgesehen sein. Das Vorsehen eines Filters im Bereich der Auslassöffnung 16 hat den Vorteil, dass dieser über die gegenüber der Auslassöffnung 16 angeordnete Einfüllöffnung 18 leicht ausgewechselt und gereinigt werden kann. Der Filter ist derart ausgebildet, dass Partikel bis zu einer Größe von mehr als etwa 0,1 mm, vorzugsweise mehr als 0,02 mm Durchmesser ausgefiltert werden.

Um zu verhindern, dass Staub oder andere Partikel in den Vorratsbehälter 10 gelangen, kann ferner in den Einlassöffnungen 24 ein entsprechender Partikelfilter vorgesehen sein.

Das Rohr 30 ist mit einem Mischbehälter 32 verbunden, wobei das Ende 34 des Rohrs 30 in den Mischbehälter 32 hineinragt. Im dargestellten Ausführungsbeispiel ist das Rohr 30 an seinem Ende 34 mit einer vorzugsweise auswechselbaren Platte oder Scheibe 36 verschlossen. Vorzugsweise in der Mitte der Scheibe 36 ist als Dosiereinrichtung eine Austrittsöffnung 38 in Form eines kreisrunden Lochs angeordnet. Das Loch weist einen Durchmesser von vorzugsweise 0,02 - 0,1 mm auf. Hierdurch ist gewährleistet, dass die vorstehend beschriebenen geringen Mengen an Luftbehandlungsmittel 12 in den Mischbehälter 32 gelangen.

In dem Rohr 30 ist ein Absperrventil 40 mit einem Handrad 42 vorgesehen, um die Zufuhr an Luftbehandlungsmittel zu dem Mischbehälter 32 zu unterbrechen. Das Ventil 40 dient somit ausschließlich zum Abstellen der Vorrichtung. Ein entsprechendes Ventil könnte auch unmittelbar hinter der Auslassöffnung 16 des Vorratsbehälters 10 angeordnet sein.

Ferner ist in dem Rohr 30 ein feinmaschiges Sieb 44 angeordnet, das zusätzlich oder anstatt eines in der Auslassöffnung 16 vorgesehenen feinmaschigen Siebs vorgesehen ist. Die Feinmaschigkeit des Siebes 44 entspricht vorzugsweise derjenigen des in der Auslassöffnung 16 ggf. vorhandenen Siebes. Das Sieb 44 ist nach dem Verschließen des Ventils 40 auswechselbar, so dass das Sieb 44 auf einfache Weise gereinigt werden kann.

Aufgrund des Höhenunterschiedes zwischen dem Vorratsbehälter 10 und der Austrittsöffnung 38 der Dosiereinrichtung ist keine Pumpe o. dgl. zum Fördern des Luftbehandlungsmittels 12 erforderlich. Die Zufuhr des Luftbehandlungsmittels 12 in den Mischbehälter 32 erfolgt ausschließlich aufgrund der Schwerkraft. Hierbei beträgt der maximale Höhenunterschied zwischen der Austrittsöffnung 38 und einer maximalen Füllhöhe des Vorratsbehälters 10 zwischen 5 und 15 cm, insbesondere zwischen 5 und 10 cm. Hierdurch ist sichergestellt, dass das Luftbehandlungsmittel 12 an der Austrittsöffnung 38 nur einen geringfügig höheren Druck als der Umgebungsdruck aufweist. Die Zufuhr des Luftbehandlungsmittels 12 zu dem Mischbehälter 32 ist somit quasi drucklos. Um einen möglichst geringen Druckunterschied zwischen vollständig befülltem Vorratsbehälter 10 und annähernd leerem Vorratsbehälter 10 zu verwirklichen, beträgt der maximale Höhenunterschied zwischen einem maximalen und einem minimalen Füllstand an Luftbehandlungsmittel 12 in dem Vorratsbehälter 10 vorzugsweise weniger als 6 cm, insbesondere weniger als 4 cm. Die Größe des Behälters ist so gewählt, dass er ein Volumen von etwa 300 - 400 ml aufweist.

Da die Zufuhr des Luftbehandlungsmittels 12 zu dem Mischbehälter 32 ausschließlich aufgrund der Schwerkraft erfolgt, ist keine Steuerung, insbesondere kein Durchflussregler o. dgl. bei dieser bevorzugten Ausführungsform erforderlich.

Innerhalb des Mischbehälters 32 ist eine Verdunstungseinrichtung 46 angeordnet. Die Verdunstungseinrichtung 46 bildet in der dargestellten Ausführungsform den Boden des Mischbehälters 32, so dass der gesamte Boden des Mischbehälters 32 als Verdunstungseinrichtung 46 ausgebildet ist. Die Verdunstungseinrichtung 46 weist eine aus einem gut wärmeleitfähigem Material bestehende Verdunstungsschale 48 auf. Die Verdunstungsschale ist vorzugsweise aus Aluminium. An der Unterseite der Verdunstungsschale, vorzugsweise an deren Außenseite ist eine Heizfolie 50 vorgesehen. Die Heizfolie 50 ist mit einer Energiequelle verbunden. Vorzugsweise wird die Heizfolie 50 mit einer 12 Volt-Energiequelle gespeist und hat eine Leistungsaufnahme von 10 - 15 Watt. Durch die Heizfolie wird die Aufnahmeschale 48 vorzugsweise auf 40 - 70, insbesondere 50 - 60 °C erwärmt. Das aus der Auslassöffnung 38 austretende Luftbehandlungsmittel verdunstet somit relativ langsam in der Aufnahmeschale 48 und steigt in Richtung einer Auslassöffnung 52 des Mischbehälters 32 auf. Vorzugsweise ist die Aufnahmeschale 48 ausgehend von der Auslassöffnung 38 fallend geneigt, so dass sich das der Aufnahmeschale 48 zugeführte Luftbehandlungsmittel über den erwärmten Boden der Aufnahmeschale 48 verteilt und gleichmäßig verdunstet.

In der Seitenwand des vorzugsweise rotationssymmetrischen Mischbehälters 32 sind Lufteintrittsöffnungen 54 vorgesehen. Die Lufteintrittsöffnungen 54 sind vorzugsweise gleichmäßig um den Umfang verteilt, um eine gleichmäßige Zufuhr von Umgebungsluft in den Mischbehälter 32 zu gewährleisten. Die Luftzufuhr durch die Lufteintrittsöffnungen 54 in den Mischbehälter 32 wird durch ein Mittel zur Erzeugung eines Luftstroms wie beispielsweise einen Ventilator 56 realisiert. Der Ventilator 56 ist gegenüber der Verdampfungseinrichtung 46 an der Austrittsöffnung 52 des Mischbehälters 32 angeordnet. Durch den Ventilator wird Luft durch die Lufteintrittsöffnungen 54 in Richtung der Pfeile 58 in den Innenraum des Mischbehälters 32 eingesaugt und in Richtung der Pfeile 60 in den zu behandelnden Raum abgegeben.

Insbesondere durch die trichterförmige Ausgestaltung des Mischbehälters 32, dessen Querschnitt sich von der Verdampfungseinrichtung 46 in Richtung der Austrittsöffnung 52 vergrößert, entsteht ein Kamineffekt innerhalb des Mischbehälters 32. Um die Verdampfung des Luftbehandlungsmittels in der Aufnahmeschale 48 nicht zu beeinträchtigen, sind die Lufteintrittsöffnungen 54 in einem Abstand zu der Verdampfungseinrichtung 46, d. h. oberhalb der Aufnahmeschale 48 angeordnet. Der Abstand der am nächsten zu der Verdampfungseinrichtung 46 vorgesehenen Lufteintrittsöffnungen 54 beträgt vorzugsweise 2 bis 8 cm, insbesondere 4-6 cm.

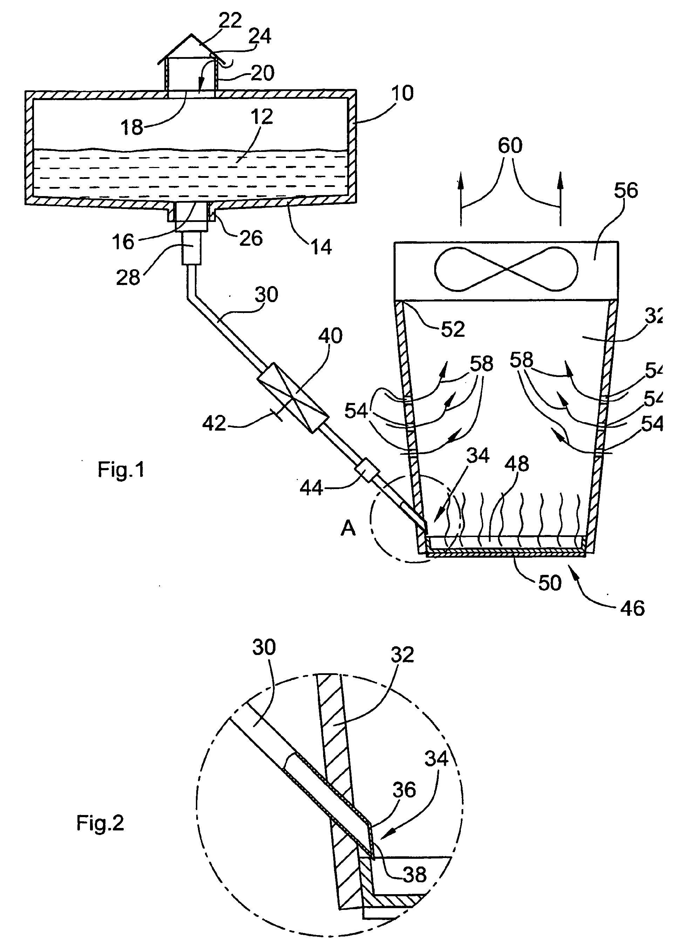

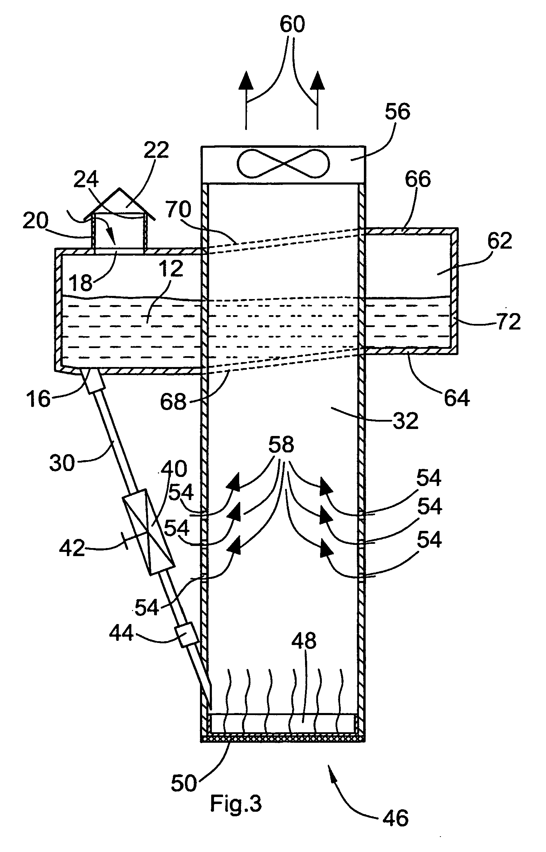

Die in Fig. 3 dargestellte zweite bevorzugte Ausführungsform entspricht prinzipiell der in den Fig. 1 und 2 dargestellten Ausführungsform. Identische oder ähnliche Bauteile der Vorrichtung sind daher mit denselben Bezugszeichen bezeichnet.

Der wesentliche Unterschied zwischen den beiden bevorzugten Ausführungsformen besteht darin, dass der erforderliche Bauraum der zweiten bevorzugten Ausführungsform (Fig. 3) kleiner ist. Dies ist dadurch erreicht, dass der Mischbehälter 32 durch einen Vorratsbehälter 62 hindurchragt. Der Vorratsbehälter 62 weist daher in einem Boden 64 sowie einer gegenüberliegenden Deckenwand 66 Öffnungen 68, 70 auf. Der Mischbehälter 32 ist durch die Öffnungen 68, 70 hindurch gesteckt, so dass der Ventilator 56 oberhalb des Vorratsbehälters 10 angeordnet ist.

Diese Anordnung des Vorratsbehälters 62 bezüglich des Mischbehälters 32 hat zur Folge, dass die Auslassöffnung 16 des Mischbehälters 62 nicht mittig, sondern an einer Seite des Vorratsbehälters angeordnet ist. Die Einfüllöffnung 18, der Ansatzstutzen 20 sowie der Deckel 22 sind im Wesentlichen wiederum gegenüber der Auslassöffnung 16 angeordnet.

Der Vorratsbehälter 62 weist vorzugsweise entsprechend dem Vorratsbehälter 10 eine kreisringförmige Seitenwand 72 auf.

Im dargestellten Ausführungsbeispiel ist der Mischbehälter 32 nicht trichterförmig, sondern kreiszylinderförmig ausgebildet. Es ist jedoch auch möglich, einen trichterförmigen Mischbehälter 32 in dieser Ausführungsform vorzusehen.

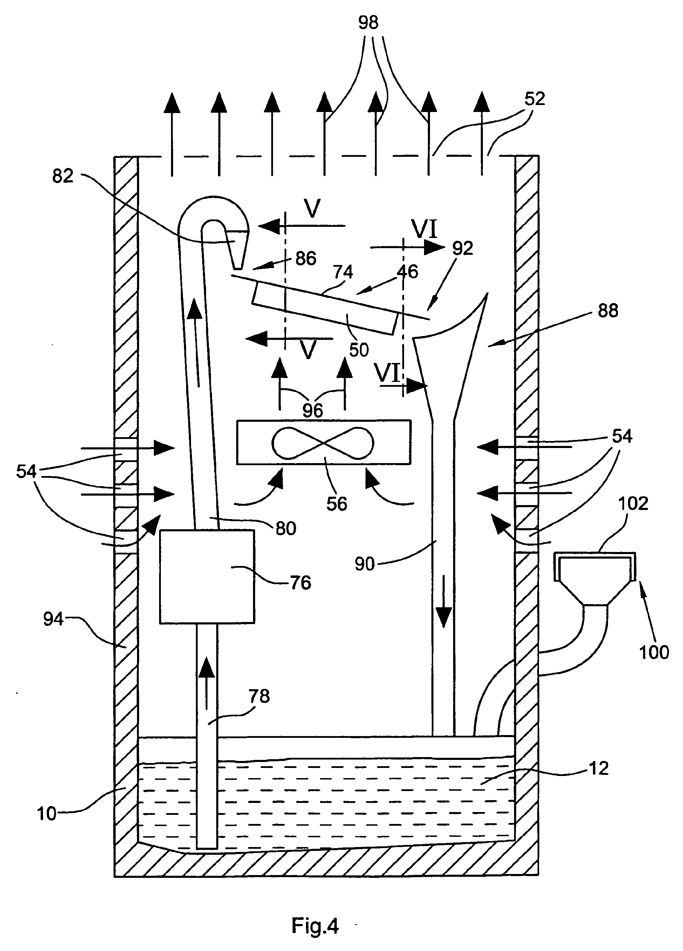

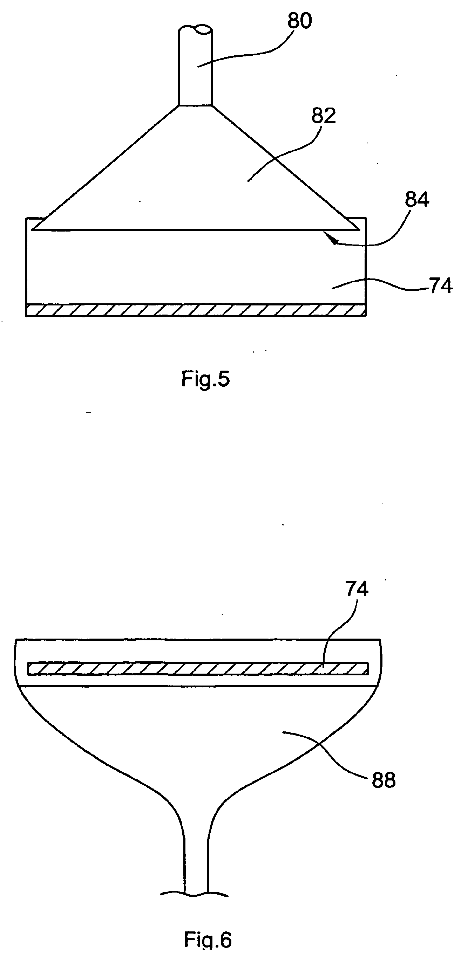

Die in Fig. 4 dargestellte Ausführungsform zeigt eine gegenüber anhand der in den Fig. 1 bis 3 beschriebenen bevorzugten Ausführungsformen der Erfindung eine weitere bevorzugte Ausführungsform mit dem wesentlichen Unterschied, dass die Verdampfungseinrichtung 46 eine gegenüber der Horizontalen geneigte Verdampfungsoberfläche 74 aufweist. Ähnliche Bestandteile, an der anhand der Fig. 4 bis 6 beschriebenen Ausführungsform der Erfindung sind mit den selben Bezugszeichen gekennzeichnet. Insbesondere hinsichtlich der Verdampfungseinrichtung 46 zugeführten Mengen sowie der Größe des Vorratsbehälters 10 entspricht diese Ausführungsform vorzugsweise im Wesentlichen den vorstehend beschriebenen Ausführungsformen.

Das Luftbehandlungsmittel 12 wird mit Hilfe einer Pumpe 76 aus einem Vorratsbehälter durch Rohrleitungen 78, 80 zu der Verdampfungseinrichtung 46 gepumpt. Das aus einer Zuführdüse 82 austretende Luftbehandlungsmittel tritt aus einer schlitzförmigen Austrittsöffnung 84 der Zuführdüse aus. Der Schlitz 84 erstreckt sich im Wesentlichen über die gesamte Breite der in dem dargestellten Ausführungsbeispiel rechteckigen Verdampfungsoberfläche 74. Bei der Verdampfungsoberfläche 74 kann es sich um ein einfaches Blech aus vorzugsweise Aluminium oder Kupfer, d. h. aus einem Material mit hoher Wärmeleitfähigkeit handeln. Das Erwärmen der Verdampfungsoberfläche 74 erfolgt durch die Heizfolie 50. Während das Luftbehandlungsmittel ausgehend von einem oberen Bereich 76 über die geneigte Verdampfungsoberfläche 74 fließt, wird aufgrund der durch die Heizfolie 50 zugeführte Wärme ein Großteil des Luftbehandlungsmittels verdampft.

Der überschüssige Teil des Luftbehandlungsmittels wird von einer Rückführeinrichtung 88 aufgefangen und über ein Rohr 90 in den Vorratsbehälter 10 zurückgeführt. Die Rückführeinrichtung 88 ist vorzugsweise trichterförmig und erstreckt sich über die gesamte Breite der Verdampfungsoberfläche 74. Die Zuführdüse 82 und die Rückführeinrichtung 88 sind somit aneinander gegenüberliegend angeordnet, wobei die Zuführdüse in dem oberen Bereich 86 der Verdunstungsoberfläche 74 und die Rückführeinrichtung 88 in einem unteren Bereich 92 der Verdampfungsoberfläche 74 angeordnet ist.

Zur Anreicherung der Luft mit Luftbehandlungsmittel weist die in den Fig. 4 bis 6 dargestellte Ausführungsform ebenfalls ein Mittel zur Erzeugung eines Luftstroms, wie einen Ventilator 56, auf. Bei der dargestellten Ausführungsform ist der Ventilator 56 unterhalb der Verdampfungseinrichtung 46 angeordnet. Die durch die Lufteintrittsöffnungen 54 in einem Mischbehälter 94 angesaugte Luft wird somit von dem Ventilator 56 in Richtung der Pfeile 96 geleitet. Da die Luft in die untere Seite der Verdampfungseinrichtung strömt, wird sie entlang der Unterseite nach außen geleitet und strömt an den Außenseiten, d. h. seitlich an der Verdampfungseinrichtung 46, vorbei nach oben in Richtung von Auslassöffnungen 52 und strömt in Richtung der Pfeile 98 in den zu behandelnden Raum ein.

Der Mischbehälter 94 dient in dem dargestellten Ausführungsbeispiel gleichzeitig als Gehäuse für die Pumpe 76, den Ventilator 56 und die Verdampfungseinrichtung 46. Ferner ist der Vorratsbehälter 10 innerhalb des als Gehäuse dienenden Behälters 94 angeordnet.

Zum Befüllen des Vorratsbehälters 10 ist ein Einfüllstutzen 100, der mit einem Deckel 102 verschließbar ist, vorgesehen.

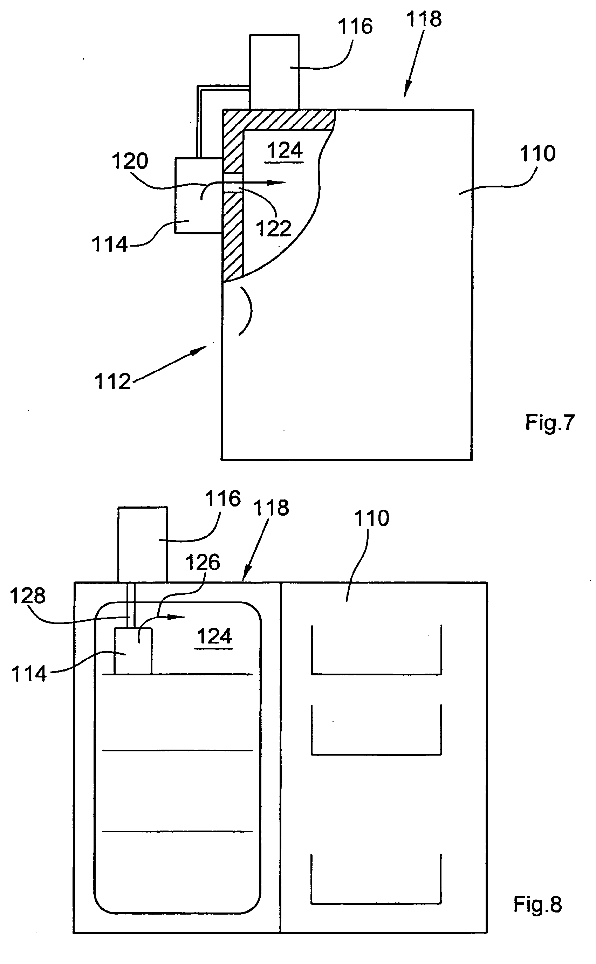

Fig. 7 zeigt eine Kühleinrichtung 110 mit einer Außenwand 112, an der eine erfindungsgemäße Vorrichtung 114 zur Luftanreicherung befestigt ist. Bei der Vorrichtung 114 handelt es sich beispielsweise um die in den Figuren 1 bis 6 beschriebenen Ausführungsformen der Erfindung. Vorzugsweise ist anstatt eines Vorratsbehälters 10 (z.B. Fig. 1 und Fig. 4) ein gesonderter Vorratsbehälter 116 vorgesehen, der beispielsweise auf einer Oberseite 118 des Kühlschranks angeordnet werden kann. Bei dem Vorratsbehälter 116 handelt es sich um einen Vorratsbehälter, dessen Volumen derart gewählt ist, das vorzugsweise nur im Abstand von ca. 6 und bevorzugt von ca. 12 Monaten Luftbehandlungsmittel nachgefüllt werden muss.

Die von der Vorrichtung 114 abgegebene mit Luftbehandlungsmittel angereicherte Luft gelangt in Richtung eines Pfeils 120 durch einen Kanal 122 in einen Innenraum 124 des Kühlschranks 110.

Bei einer weiteren bevorzugten Ausführungsform der Erfindung (Fig. 8) ist die Vorrichtung zur Luftanreicherung 114 innerhalb des Innenraums 124 des Kühlschranks 110 angeordnet. Bei der Vorrichtung 114 handelt es sich vorzugsweise um eine der vorstehend beschriebenen bevorzugten Ausführungsformen der Vorrichtung. Das Ausbringen der Luftbehandlungsmittel aufweisenden Luft erfolgt unmittelbar in den Kühlschrankinnenraum 124 in Richtung eines Pfeils 126.

Entsprechend der in Fig. 1-6 beschriebenen Ausführungsformen kann ein Vorratsbehälter innerhalb des Kühlschranks 110 angeordnet sein. Vorzugsweise wird jedoch ein größerer Vorratsbehälter 116 auf einer Oberseite 118 des Kühlschranks angeordnet. Der Vorratsbehälter 116 ist über einen Verbindungsschlauch 128 mit der Vorrichtung 114 verbunden. Weiterhin kann die Vorrichtung 114 zusätzlich einen eigenen Vorratsbehälter aufweisen, der ein kleineres Volumen als der Vorratsbehälter 116 aufweist und beispielsweise eine Zwischenkammer oder dergleichen automatisch von dem größeren Vorratsbehälter 116 gefüllt wird. Die Vorrichtung 116 dient somit zum Nachfüllen einer innerhalb der Vorrichtung 114 angeordneten Vorratskammer.

Vorzugsweise ist die Vorrichtung 114 von einem isolierenden Gehäuse umgeben, um ein Aufwärmen des Kühlschranks durch das Vorsehen der erfindungsgemäßen Vorrichtung 114 innerhalb des Kühlraums 124 zu vermeiden.

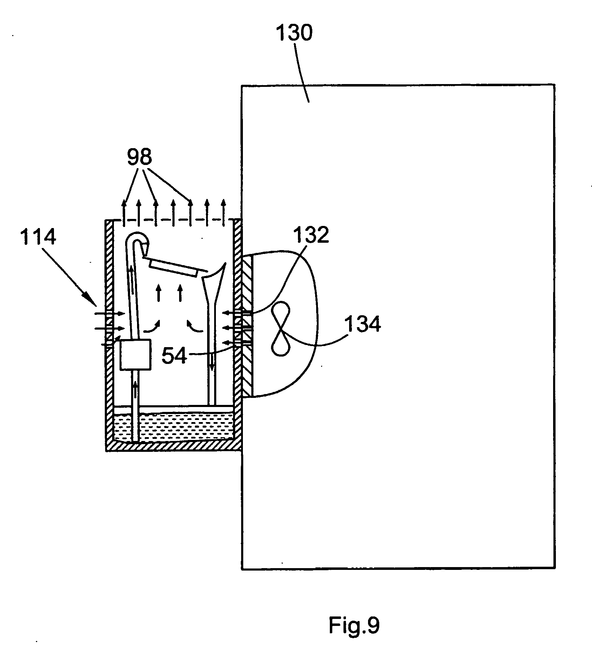

Bei einer weiteren bevorzugten Ausführungsform der Erfindung (Fig. 9) ist eine Vorrichtung zur Luftanreicherung außen an einem Gehäuse 130 eines Computers angeordnet. In dem angeführten Ausführungsbeispiel ist eine Vorrichtung entsprechend Fig. 4 vorgesehen. Selbstverständlich ist es ebenso möglich, die anhand der in Fig. 1 und 3 dargestellten Ausführungsformen oder anderer Ausführungsformen der erfindungsgemäßen Vorrichtung vorzusehen. Eine besondere Ausgestaltung der in Fig. 9 dargestellten Ausführungsform besteht darin, dass Luftaustrittsschlitze 132 des Computergehäuses 130 auf Höhe der Lufteintrittsöffnungen 54 vorgesehen sind. Durch ein Gebläse 134 des Computers erfolgt somit unmittelbar der Transport der mit Luftbehandlungsmittel versehenen Luft. Ein Ventilator 56 kann somit bei der Vorrichtung 114 entfallen. Hierdurch ist es möglich die Vorrichtung 114 innerhalb des Computergehäuses anzuordnen.

### Die Erfindung betrifft weiterhin

ein Verfahren zum Entkeimen und/oder Entstinken einer Kühleinrichtung, insbesondere zum Entkeimen eines Kühlschranks, umfassend das Verdampfen einer antimikrobiellen Zusammensetzung und/oder einer geruchsmaskierenden Zusammensetzung in der Kühleinrichtung mittels einer wie oben definierten Vorrichtung und
ein Verfahren zum Entkeimen eines Computers und des Raumes, in dem der Computer aufgestellt ist, umfassend das Verdampfen einer antimikrobiellen Zusammensetzung mittels einer wie oben definierten Vorrichtung, die im Bereich der Belüftungsanlage des Computers angeordnet ist.

Die genannten antimikrobiellen Zusammensetzungen und die geruchsmaskierenden Zusammensetzung sind dabei vorzugsweise die vorstehend definierten Zusammensetzungen (X) und (Y).

Bei dem Entkeimungsverfahren für Kühleinrichtungen wird die antimikrobielle Zusammensetzung - in Abhängigkeit des Kühlschrankvolumens - in einer Menge von 0,0001 bis 0,5 ml pro Stunde verdampft. Dabei kann die antimikrobielle Zusammensetzung noch die vorstehend definierte geruchsmaskierende Verbindung enthalten, wodurch ggf. herrschender unangenehmer Geruch im Kühlschrank verringert werden kann.

In den genannten Verfahren erfolgt das Einbringen der antimikrobiellen Zusammensetzung dergestalt, dass eine Raumluftkonzentration von kleiner gleich 100 ppb (parts per billion) vorzugsweise von etwa 10 ppb gewährleistet ist, was durch ein Einbringen von 0,01 ml/m³ Raum pro Stunde gewährleistet wird.

Die Erfindung wird anhand der folgenden Beispiele näher erläutert.

### Beispiele

In den nachfolgenden Beispielen wurde als antimikrobielle Zusammensetzung HiQ®-Caire (bestehend aus 1 Gew.-% Tannin, 0,5 Gew.-% etherisches Öl und der Rest Propylenglykol) eingesetzt.

Beispiel 1: PC-Test. Die Keime im PC und in der Raumluft werden durch den Einsatz von HiQ®-Caire durch Einbringung mit der erfindungsgemäßen Vorrichtung innerhalb des PC-Towers im Ausströmbereich des Ventilators (siehe Fig. 9) bei einer Aufbringungsmenge von 0,01 ml/m³/h (HiQ®Caire-Konzentration ≈ 10 ppb) gegen Null reduziert. Die Versuchsergebnisse sind in Tabelle 1 dargestellt.

**Tabelle 1**

| Probenahme/Volumen(I) | 500 | Schimmel* Keimzahl pro m³ | | | |
|---|---|---|---|---|---|
| | | 100 | 50 | 20 | 10 |
| vor Kontamin. | 58 | n. u.** | n. u. | n. u. | n. u. |
| gleich nach Kontamin. | n. u. | 47.400 | >500.000 | >500.000 | >500.000 |
| nach 1 Tag | 4 | 0 | 0 | n. u. | 0 |
| nach 2 Tgane | 2 | 0 | 0 | n. u. | n. u. |
| nach 3 Tagen | 0 | 0 | 0 | n. u. | n. u. |
| nach 4 Tagen | 0 | n. u. | n. u. | n. u. | n. u. |

| | | | | | |
|---|---|---|---|---|---|
| * Schimmel = Aspergillus und Penicilium | | | | | |
| ** n. u. = nicht untersucht. | | | | | |

Beispiel 2: Kühlschranktest. 0,5 ml pro h von HiQ-Caire wurde mittels einer erfindungsgemäßen Verdampfungsvorrichtung von außen in den Kühlschrank eingebracht (siehe Fig. 7). Dadurch wurden die Keime auf den Lebensmitteln im Kühlschrank signifikant reduziert. Die Versuchsergebnisse sind in Tabelle 2 dargestellt.

**Tabelle 2**

| | Reis (Vergleichstest) | Reis mit HiQ® |
|---|---|---|
| Gesamtkeimzahl KbE/g | 70 | 70 |
| 3. Untersuchungstag | 100 | 100 |
| 6. Untersuchungstag | 1,3 x 10⁵ | <10 |
| 8. Untersuchungstag | 6,4 x 10⁵ | <10 |

## Patentansprüche

1. Vorrichtung zur Anreicherung von Luft mit einem Luftbehandlungsmittel, mit
einem Vorratsbehälter (10, 62) zur Aufnahme von flüssigem Luftbehandlungsmittel (12),
einer mit dem Vorratsbehälter (10, 62) verbundenen Verdampfungseinrichtung (46) zum Erwärmen des Luftbehandlungsmittels (12),
einer zwischen dem Vorratsbehälter (10, 62) und der Verdampfungseinrichtung (46) angeordneten Dosiereinrichtung (38) zur mengenbegrenzten Zufuhr von flüssigem Luftbehandlungsmittel (12) zu der Verdampfungseinrichtung (46),
einem mit der Verdampfungseinrichtung (46) verbundenen, von Luft durchströmten Mischbehälter (32) zum Einmischen von dampfförmigen Luftbehandlungsmittel in die Luft und
einem dem Mischbehälter (32) zugeordneten Mittel (56) zur Erzeugung eines Luftstroms, das Luft durch eine Lufteintrittsöffnung (54) ansaugt und ein Gemisch aus Luft und dampfförmigen Luftbehandlungsmittel aus einer Austrittsöffnung abgibt,
wobei das durch die Austrittsöffnung (52) des Mischbehälters (32) abgegebene Gemisch aus Luft und dampfförmigen Luftbehandlungsmittel pro Kubikmeter Luft einen Luftbehandlungsmittel-Anteil von 0,00001 ml bis 0,5 ml, vorzugsweise 0,0001 bis 0,01 ml aufweist.

2. Vorrichtung nach Anspruch 1, **dadurch gekennzeichnet, dass** die Dosiereinrichtung eine Austrittsöffnung (38) aufweist, deren Querschnitt kleiner gleich 0,0078 mm² insbesondere kleiner gleich 0,00031 mm² ist.

3. Vorrichtung nach Anspruch 1 oder 2, **dadurch gekennzeichnet, dass** zwischen dem Vorratsbehälter (10, 62) und der Verdampfungseinrichtung (46) ein Höhenunterschied besteht, so dass die Zufuhr von Luftbehandlungsmitteln (12) ausschließlich durch Schwerkraft erfolgt.

4. Vorrichtung nach einem der Ansprüche 1-3, **dadurch gekennzeichnet, dass** die Dosiereinrichtung ein Fördermittel zum Fördern des Luftbehandlungsmittels (12) zu dem Mischbehälter (32) aufweist.

5. Vorrichtung zur Anreicherung von Luft mit einem Luftbehandlungsmittel, mit
einem Vorratsbehälter (10) zur Aufnahme von flüssigem Luftbehandlungsmittel (12),
einer mit dem Vorratsbehälter (10) verbundenen Verdampfungseinrichtung (46) zum Erwärmen des Luftbehandlungsmittels (12), und
einer Rückführeinrichtung (88) zum Zurückführen von überschüssigem Luftbehandlungsmittel (12) von der Verdunstungseinrichtung (46) in den Vorratsbehälter (10),
wobei die Verdampfungseinrichtung (46) eine geneigt angeordnete Verdampfungsoberfläche (74) aufweist, über die das zu verdampfende Luftbehandlungsmittel (12) fließt.

6. Vorrichtung nach Anspruch 5, **dadurch gekennzeichnet, dass** die Verdampfungsoberfläche (74) eine Neigung gegenüber einer Horizontalen von 10° - 30°, vorzugsweise 15° - 25° aufweist.

7. Vorrichtung nach Anspruch 5 oder 6, **dadurch gekennzeichnet, dass** die Verdampfungsoberfläche (76) in Fließrichtung verlaufende Transportrillen aufweist.

8. Vorrichtung nach einem der Ansprüche 5-7, **dadurch gekennzeichnet, dass** die Verdampfungseinrichtung (46) eine Zuführdüse (82) zum gleichmäßigen Verteilen des Luftbehandlungsmittels auf der Verdampfungsoberfläche (76) aufweist, wobei sich die Zuführdüse (82) vorzugsweise im Wesentlichen über die gesamte Breite der Verdampfungsoberfläche (74) erstreckt.

9. Vorrichtung nach einem der Ansprüche 5 - 8, **dadurch gekennzeichnet, dass** die Rückführeinrichtung (88) eine vorzugsweise trichterförmige Auffangeinrichtung aufweist und vorzugsweise gegenüber der Zuführdüse (82) angeordnet ist.

10. Vorrichtung nach einem der Ansprüche 5-9, **gekennzeichnet durch** einen mit der Verdampfungseinrichtung (46) verbundenen, von Luft durchströmten Mischbehälter (94) zum Einmischen von dampfförmigem Luftbehandlungsmittel in die Luft.

11. Vorrichtung nach einem der Ansprüche 5 - 10, **gekennzeichnet durch** ein Mittel (56) zur Erzeugung eines Luftstroms, wobei das Mittel (56) zur Erzeugung des Luftstroms unterhalb der Verdunstungseinrichtung (46) angeordnet ist, so dass Luft seitlich an der Verdunstungseinrichtung vorbei strömt.

12. Vorrichtung nach einem der Ansprüche 1 - 11, **dadurch gekennzeichnet, dass** die Verdampfungseinrichtung (46) in einem Bodenbereich des Mischbehälters (32) und die Austrittsöffnung (52) gegenüber der Verdampfungseinrichtung (46) zur Erzeugung eines Kamineffekts angeordnet ist.

13. Vorrichtung nach einem der Ansprüche 1 - 12, **dadurch gekennzeichnet, dass** die Verdampfungseinrichtung (46) innerhalb des Mischbehälters (32) angeordnet ist.

14. Vorrichtung nach Anspruch 12 oder 13, **dadurch gekennzeichnet, dass** die Lufteintrittsöffnung (54) in einer Seitenwand des Mischbehälters (32) oberhalb der Verdampfungseinrichtung (46) angeordnet ist.

15. Vorrichtung nach einem der Ansprüche 1 - 14, **dadurch gekennzeichnet, dass** der Mischbehälter (32) in Richtung der Austrittsöffnung (52) trichterförmig ausgebildet ist.

16. Vorrichtung nach einem der Ansprüche 12 - 15, **dadurch gekennzeichnet, dass** die Querschnittsfläche der Lufteintrittsöffnung (54) größer als die Querschnittsfläche der Austrittsöffnung (52) ist.

17. Vorrichtung nach einem der Ansprüche 1 - 16, **dadurch gekennzeichnet, dass** der Vorratsbehälter (10, 62) eine Druckausgleichseinrichtung (24) aufweist, so dass im Vorratsbehälter (10, 62) Umgebungsdruck herrscht, wobei die Druckausgleichseinrichtung (24) vorzugsweise einen Partikelfilter aufeist.

18. Vorrichtung nach einem der Ansprüche 1 - 17, **dadurch gekennzeichnet, dass** die Verdampfungseinrichtung (46) eine Aufnahmeschale (48) aus Material mit guter Leitfähigkeit zur Aufnahme von Luftbehandlungsmittel (12) aufweist und mit der Aufnahmeschale (48) eine Heizeinrichtung (50), insbesondere eine Heizfolie verbunden ist.

19. Vorrichtung nach einem der Ansprüche 1 - 18, **dadurch gekennzeichnet, dass** der Dosiereinrichtung (38) ein feinmaschiges Sieb (44) vorgeschaltet ist.

20. Vorrichtung nach einem der Ansprüche 1 - 19, **dadurch gekennzeichnet, dass** in dem zu behandelnden Raum zugeführtes Gemisch aus Luft und Luftbehandlungsmittel der Luftbehandlungsmittel-Anteil kleiner gleich 500 ppb, vorzugsweise kleiner als 10ppb ist.

21. Vorrichtung nach einem der Ansprüche 1 - 20, **dadurch gekennzeichnet, dass** als Luftbehandlungsmittel eine antimikrobielle Zusammensetzung verwendet wird, die vorzugsweise einen oder mehrere GRAS-Aromastoffe oder deren Derivate enthält.

22. Vorrichtung nach einem der Ansprüche 1 - 21, **dadurch gekennzeichnet, dass** als Luftbehandlungsmittel eine geruchsmaskierende Zusammensetzung verwendet wird, die wenigstens eine geruchmaskierende Komponente (A) ausgewählt aus Terpenen, Maisstärke, Mangansalzen, ätherischen Ölen und Poyvinylpyrrolidon mit vorzugsweise einer Molmasse von 10.000 bis 60.000, vorzugsweise 30.000 bis 50.000 enthält.

23. Vorrichtung nach Anspruch 22, wobei die geruchsmaskierte Zusammensetzung weiterhin eine Functional-Flavour-Komponente (B) enthält.

24. Vorrichtung nach Anspruch 23, **dadurch gekennzeichnet, dass** die Functonal-Flavour-Komponente (B) eine, vorzugsweise mehrere, der folgenden Substanzen enthält:
Hexlbutyrat, Octylacetat, Isobutylisobutyrat, cis-3-Hexen-1-ylacetat cis-3-γ -Decalactone, Ethylcaproat, Butylacetat, Ethylbenzoat, Ethylbutyrat, Hexylacetat, Methylcaproat, Phenylethylalkohol, Citonellol, Undecylaldehyd, Benzylphenylacetat, Cinnamik-Alkohl, Eugenol, Benzylacetat, Linalcool, cis-Jasmone, Acetylmethylanthranilat, cis-3-Hexen-1-ol, cis-3-Hexen-1-ylsalicyclat, Methylbenzoat, Methylsalicylat, Geranylacetat, cis-3-Hexen-1-ylacetat, Litsea Cubeba, Orangenöl, Phenylpropylalkohol und Phenylethylacetat.

25. Kühleinrichtung, insbesondere Haushaltskühlschrank, mit einer Vorrichtung zur Luftanreichung nach einem der Ansprüche 1 bis 24.

26. Kühleinrichtung nach Anspruch 25, **dadurch gekennzeichnet, dass** die Vorrichtung (114) zur Luftanreicherung an einem Kühleinrichtungsgehäuse angeordnet ist und die Vorrichtung zur Luftanreicherung über einen Kanal (122) der mit einem Kühleinrichtungsinnenraum (124) verbunden ist.

27. Kühleinrichtung nach Anspruch 26, **dadurch gekennzeichnet, dass** die Vorrichtung (114) zur Luftanreicherung im Kühleinrichtungsinnenraum (124) angeordnet ist.

28. Computer mit einer Vorrichtung (114) zur Luftanreicherung nach einem der Ansprüche 1 - 24, wobei die Vorrichtung (114) zur Luftanreicherung vorzugsweise im Bereich einer Computerbelüftungseinrichtung (134) angeordnet ist.

29. Computer nach Anspruch 28, **dadurch gekennzeichnet, dass** das Fördern des Luftbehandlungsmittels durch die Computerbelüftungseinrichtung (134) erfolgt.

30. Computer nach einem der Ansprüche 28 oder 29, **dadurch gekennzeichnet, dass** die vom Computer erzeugte Wärme als Verdunsteinrichtung dient.

31. Verfahren zum Entkeimen und/ oder Entstinken einer Kühleinrichtung, insbesondere zum Entkeimen eines Kühlschranks, umfassend des Verdampfen einer antimikrobiellen Zusammensetzung und/ oder einer geruchsmaskierenden Zusammensetzung in der Kühleinrichtung mittels einer wie in Ansprüchen 1-24 definierten Vorrichtung.

32. Verfahren zum Entkeimen eines Computers und des Raumes, in dem der Computer aufgestellt ist, umfassend des Verdampfen einer antimikrobiellen Zusammensetzung mittels einer wie in Ansprüchen 1 - 24 definierten Vorrichtung, die im Bereich der Belüftungsanlage des Computers angeordnet ist.

33. Verfahren nach Anspruch 31 oder 32, wobei die antimikrobielle Zusammensetzung einen oder mehrere GRAS-Aromastoffe oder deren Derivate enthält.

34. Verfahren nach Anspruch 32 oder 33, wobei zum Entkeimen eines Kühlschrankes die antimikrobielle Zusammensetzung in einer Menge von 0,01 bis 0,5 ml/h verdampft wird.

35. Verfahren nach einem der Ansprüche 31 - 34, wobei die Konzentration der antimikrobiellen Zusammensetzung in der Kühleinrichtung, dem Computer oder den Raum, in der der Computer aufgestellt ist, kleiner gleich 500 ppb, vorzugsweise kleiner als 10 ppb ist.

## Claims

1. A device for enriching air with an air treatment agent, comprising:
a storage vessel (10, 62) for receiving liquid air treatment agent (12);
an evaporating means (46) for heating the air treatment agent (12), connected with the storage vessel (10, 62);
a dosing means (38) provided between said storage vessel (10, 62) and said evaporating means (46) for the quantity-restricted supply of liquid air treatment agent (12) to said evaporating means (46); and
a mixing vessel (32) which is connected with said evaporating means (46) and through which air is flowing, for admixing vaporized air treatment agent into the air, and
a means (56) for generating a current of air which sucks air in through the air inlet opening (54) and exhausts a mixture of air and vaporized air treatment agent out through an outlet, said means being assigned to the mixing vessel (32),
wherein the mixture of air and vaporized air treatment agent exhausted through the outlet (52) of the mixing vessel (32) has a content of air treatment agent per cubic meter of air of from 0.00001 ml to 0.5 ml, preferably from 0.0001 to 0.01 ml.

2. The device according to claim 1, **characterized in that** said dosing means has an outlet (38) whose cross-sectional area is smaller than or equal to 0.0078 mm², especially smaller than or equal to 0.00031 mm².

3. The device according to claim 1 or 2, **characterized in that** the storage vessel (10, 62) and the evaporating means (46) are at different levels so that the supply of air treatment agent (12) is effected exclusively by gravity.

4. The device according to any of claims 1 to 3, **characterized in that** said dosing means comprises a conveying means for conveying said air treatment agent (12) into said mixing vessel (32).

5. A device for enriching air with an air treatment agent, comprising:
a storage vessel (10) for receiving liquid air treatment agent (12);
an evaporating means (46) for heating the air treatment agent (12), connected with the storage vessel (10); and
a recirculating means (88) for recirculating excess air treatment agent (12) from the evaporating means (46) into the storage vessel (10);
wherein said evaporating means (46) has an inclined evaporation surface (74) over which the air treatment agent (12) to be evaporated flows.

6. The device according to claim 5, **characterized in that** said evaporation surface (74) has an inclination of from 10° to 30°, preferably from 15 to 25°, relative to a horizontal plane.

7. The device according to claim 5 or 6, **characterized in that** said evaporation surface (74) has conveying grooves running in the direction of flow.

8. The device according to any of claims 5 to 7, **characterized in that** said evaporating means (46) has a feeding nozzle (82) for uniformly distributing the air treatment agent on the evaporation surface (76), said feeding nozzle (82) essentially extending over the entire width of the evaporation surface (74).

9. The device according to any of claims 5 to 8, **characterized in that** said recirculating means (88) has a collecting means, which is preferably funnel-shaped, said recirculating means (88) preferably being arranged opposite said feeding nozzle (82).

10. The device according to any of claims 5 to 9, **characterized by** a mixing vessel (94) which is connected with said evaporating means (46) and through which air is flowing, for admixing vaporized air treatment agent into the air.

11. The device according to any of claims 5 to 10, **characterized by** an agent (56) for generating an air current, wherein said agent (56) for generating an air current is arranged below the evaporating means (46), so that air flows laterally past the evaporating means.

12. The device according to any of claims 1 to 11, **characterized in that** said evaporating means (46) is arranged in a bottom zone of the mixing vessel (32), and the outlet (52) is arranged opposite the evaporating means (46), to produce a stack effect.

13. The device according to any of claims 1 to 12, **characterized in that** said evaporating means (46) is arranged within said mixing vessel (32).

14. The device according to any of claims 12 or 13, **characterized in that** said air inlet opening (54) is provided in a lateral wall of said mixing vessel (32) above said evaporating means (46).

15. The device according to any of claims 1 to 14, **characterized in that** said mixing vessel (32) has a funnel-shaped design in the direction of the outlet (52).

16. The device according to any of claims 12 to 15, **characterized in that** the cross-sectional area of the air inlet opening (54) is greater than the cross-sectional area of the outlet (52).

17. The device according to any of claims 1 to 16, **characterized in that** said storage vessel (10, 62) has a pressure compensation means (24) so that ambient pressure prevails in the storage vessel (10, 62), said pressure compensation means (24) preferably comprising a particle filter.

18. The device according to any of claims 1 to 17, **characterized in that** said evaporating means (46) comprises a receptor dish (48) made of a material having a good heat conductivity for receiving air treatment agent (12), and a heating means (50), especially a heating film, is connected with said receptor dish (48).

19. The device according to any of claims 1 to 18, **characterized in that** a fine-meshed sieve (44) is incorporated prior to said dosing means (38).

20. The device according to any of claims 1 to 19, **characterized in that** the content of air treatment agent in the mixture of air and air treatment agent supplied to the space to be treated is smaller than or equal to 500 ppb, preferably smaller than 10 ppb.

21. The device according to any of claims 1 to 20, **characterized in that** an antimicrobial composition is used as said air treatment agent, said antimicrobial composition preferably containing one or more GRAS flavoring agents or their derivatives.

22. The device according to any of claims 1 to 21, **characterized in that** an odor-masking composition which contains at least one odor-masking component (A) selected from terpenes, corn starch, manganese salts, essential oils and polyvinyl pyrrolidone having a molecular weight of from 10,000 to 60,000, preferably from 30,000 to 50,000, is used as said air treatment agent.

23. The device according to claim 22, wherein said odor-masking composition further contains a functional flavor component (B).

24. The device according to claim 23, **characterized in that** said functional flavor component (B) contains one, preferably several, of the following substances:
hexyl butyrate, octyl acetate, isobutyl isobutyrate, cis-3-hexene-1-yl acetate, γ-decalactone, ethyl caproate, butyl acetate, ethyl benzoate, ethyl butyrate, hexyl acetate, methyl caproate, phenylethyl alcohol, citronellol, undecyl aldehyde, benzylphenyl acetate, cinnamyl alcohol, eugenol, benzyl acetate, linalool, cis-jasmone, acetylmethyl anthranilat, cis-3-hexene-1-ol, cis-3-hexene-1-yl salicylate, methyl benzoate, methyl salicylate, geranyl acetate, cis-3-hexene-1-yl acetate, Litsea cubeba, orange oil, phenylpropyl alcohol and phenylethyl acetate.

25. A refrigerating means, especially a household refrigerator, comprising a device for enriching air according to any of claims 1 to 24.

26. The refrigerating means according to claim 25, **characterized in that** said device (114) for enriching air is provided at a housing of a refrigerating means and said device for enriching air is connected with the interior space (124) of the refrigerating means through a duct (122).

27. The refrigerating means according to claim 26, **characterized in that** said device (114) for enriching air is provided in the interior space (124) of the refrigerating means.

28. A computer comprising a device (114) for enriching air according to any of claims 1 to 24, said device (114) for enriching air being provided in the region of a computer aeration means (134).

29. The computer according to claim 28, **characterized in that** the conveying of the air treatment agent is effected by said computer aeration means (134).

30. The computer according to any of claims 28 or 29, **characterized in that** the heat generated by the computer serves as said evaporating means.

31. A method for sterilizing and/or deodorizing a refrigerating means, especially for sterilizing a refrigerator, comprising evaporation of an antimicrobial composition and/or an odor-masking composition within the refrigerating means using a device as defined in claims 1 to 24.

32. A method for sterilizing a computer and the room where the computer is installed, comprising evaporation of an antimicrobial composition using a device as defined in claims 1 to 24 provided in the region of the aeration system of the computer.

33. The method according to claim 31 or 32, wherein said antimicrobial composition contains one or more GRAS flavoring agents or their derivatives.

34. The method according to claim 32 or 33, wherein said antimicrobial composition is evaporated in an amount of from 0.01 to 0.5 ml/h for sterilizing a refrigerator.

35. The method according to any of claims 31 to 34, wherein the concentration of the antimicrobial composition in the refrigerating means, the computer or the room where the computer is installed is smaller than or equal to 500 ppb, preferably smaller than 10 ppb.

## Revendications

1. Dispositif pour traiter l'air avec un agent de traitement de l'air, comprenant
un réservoir de stockage (10, 62), pour recevoir de l'agent de traitement de l'air (12) liquide,
un dispositif d'évaporation (46) relié au réservoir de stockage (10, 62), pour chauffer l'agent de traitement de l'air (12),
un dispositif de dosage (38) disposé entre le réservoir de stockage (10, 62) et le dispositif d'évaporation (46) pour effectuer un apport, en quantité limitée, d'un agent de traitement de l'air (12) liquide au dispositif d'évaporation (46),
un récipient de mélange (32) traversé par un écoulement d'air, relié au dispositif d'évaporation (46), pour mettre en mélange des agents de traitement de l'air, présents dans l'air et se présentant sous forme de vapeur, et
un moyen (56) associé au récipient de mélange (32) pour produire un écoulement d'air, aspirant de l'air à travers une ouverture d'entrée d'air (54) et délivrant, depuis une ouverture de sortie, un mélange formé d'air et d'agent de traitement de l'air se présentant sous forme de vapeur,
le mélange, délivré par l'ouverture de sortie (52) du réservoir de mélange (32), mélange formé d'air et d'agent de traitement de l'air se présentant sous forme de vapeur, présentant par mètre cube d'air une proportion d'agent de traitement de l'air de 0,00001 ml à 0,5 ml, de préférence de 0,001 à 0,01 ml.

2. Dispositif selon la revendication 1, **caractérisé en ce que** le dispositif de dosage présente une ouverture de sortie (38), dont la section transversale est inférieure ou égale à 0,0078 mm², en particulier inférieure ou égale à 0,00031 mm².

3. Dispositif selon la revendication 1 ou 2, **caractérisé en ce que**, entre le récipient de stockage (10, 62) et le dispositif d'évaporation (46), existe une différence de niveau, de sorte que l'apport d'agents de traitement de l'air (32) s'effectue exclusivement sous la force de la gravité.

4. Dispositif selon l'une des revendications 1 à 3, **caractérisé en ce que** le dispositif de dosage présente un moyen de transport pour transporter l'agent de traitement de l'air (12) au récipient de mélange (32).

5. Dispositif pour traiter l'air avec un agent de traitement de l'air, comprenant :
un récipient de stockage (10), pour recevoir un agent de traitement de l'air (12) liquide,
un dispositif d'évaporation (46) relié au récipient de stockage (10), pour chauffer l'agent de traitement de l'air (12),
un dispositif de recyclage (88), pour recycler l'excès d'air de traitement (12) issu du dispositif d'évaporation (46), dans le réservoir de stockage (10),
où le dispositif d'évaporation (46) présente une surface d'évaporation (76) inclinée, sur laquelle s'écoule l'agent de traitement de l'air (12) à évaporer.

6. Dispositif selon la revendication 5, **caractérisé en ce que** la surface d'évaporation (74) présente, par rapport à l'horizontale, une inclinaison de 10° à 30°, de préférence de 15° à 25°.

7. Dispositif selon la revendication 5 ou 6, **caractérisé en ce que** la surface d'évaporation (76) présente des cannelures de transport s'étendant dans la direction d'écoulement.

8. Dispositif selon l'une des revendications 5 à 7, **caractérisé en ce que** le dispositif d'évaporation (46) présente une buse d'amenée (82) servant à distribuer régulièrement l'agent de traitement de l'air sur la surface d'évaporation (76), la buse d'amenée (82) s'étendant de préférence sensiblement sur toute la largeur de la surface d'évaporation (74).

9. Dispositif selon l'une des revendications 5 à 8, **caractérisé en ce que** le dispositif de re-circulation (88) présente un dispositif de captation, de préférence en forme d'entonnoir, et est disposé de préférence à l'opposé de la buse d'amenée (82).

10. Dispositif selon l'une des revendications 5 à 9, **caractérisé par** un récipient de mélange (94) relié au dispositif d'évaporation (46), parcouru par un écoulement d'air, pour la mise en mélange, dans l'air, de l'agent de traitement de l'air se présentant sous forme de vapeur.

11. Dispositif selon l'une des revendications 5 à 10, **caractérisé par** un moyen (56) pour produire un écoulement d'air, le moyen (56) pour produire l'écoulement d'air étant disposé au-dessous du dispositif d'évaporation (46), de sorte que l'air s'écoule en passant latéralement sur le dispositif d'évaporation.

12. Dispositif selon l'une des revendications 1 à 11, **caractérisé en ce que** le dispositif d'évaporation (46) est disposé dans une zone de fond du récipient de mélange (32) et l'ouverture de sortie (52) est disposée à l'opposé du dispositif d'évaporation (46), pour produire un effet de cheminée.

13. Dispositif selon l'une des revendications 1 à 12, **caractérisé en ce que** le dispositif d'évaporation (46) est disposé à l'intérieur du récipient de mélange (32).

14. Dispositif selon la revendication 12 ou 13, **caractérisé en ce que** l'ouverture d'entrée d'air (54) est disposée dans une paroi latérale de récipient de mélange (32), au-dessus du dispositif d'évaporation (46).

15. Dispositif selon l'une des revendications 1 à 14, **caractérisé en ce que** le récipient de mélange (32) est réalisé en forme d'entonnoir, dans la direction de l'ouverture de sortie (52).

16. Dispositif selon l'une des revendication 12 à 15, **caractérisé en ce que** l'aire de section transversale de l'ouverture d'entrée d'air (54) est plus grande que l'aire de section transversale de l'ouverture de sortie (52).

17. Dispositif selon l'une des revendications 1 à 16, **caractérisé en ce que** le récipient de stockage (10, 62) présente un dispositif d'équilibrage de pression (24), de sorte qu'une pression ambiante règne dans le récipient de stockage (62), le dispositif d'équilibrage de pression (24) présentant de préférence un filtre à particules.

18. Dispositif selon l'une des revendications 1 à 17, **caractérisé en ce que** le dispositif d'évaporation (46) présente une coque de réception (48), formée d'un matériau ayant une bonne conductivité thermique, pour recevoir l'agent de traitement de l'air (12), et un dispositif de chauffage (50), en particulier une feuille de chauffage, est relié(e) à la coque de réception (48).

19. Dispositif selon l'une des revendications 1 à 18, **caractérisé en ce qu'**en amont du dispositif de dosage (38) est installé un tamis (44) à mailles fines.

20. Dispositif selon l'une des revendications 1 à 19, **caractérisé en ce que**, dans le mélange amené à l'espace à traiter, mélange formé d'air et d'agent de traitement de l'air, la proportion d'agent de traitement de l'air est inférieure ou égale à 500 ppb, de préférence inférieure ou égale à 10 ppb.

21. Dispositif selon l'une des revendications 1 à 20, **caractérisé en ce qu'**est utilisé comme agent de traitement de l'air une composition anti-microbienne contenant de préférence une ou plusieurs substances aromatiques GRAS (Generally Recognized As Safe) ou bien leurs dérivés.

22. Dispositif selon l'une des revendications 1 à 21, **caractérisé en ce que** l'on utilise comme agent de traitement de l'air une composition masquant les odeurs, contenant au moins un composant A masquant les odeurs, sélectionné parmi les terpènes, l'amidon de maïs, les sels de manganèse, les huiles éthériques et la polyvinylpyrrolidone, avec de préférence une masse molaire de 10.000 à 60.000, de préférence de 30.000 à 50.000.

23. Dispositif selon la revendication 22, la composition masquant les odeurs contenant en plus un composant fonctionnel odoriférant B.

24. Dispositif selon la revendication 23, **caractérisé en ce que** le composant fonctionnel odoriférant B contient de préférence plusieurs des substances suivantes :
héxybutyrate, octylacétate, isobutylisobutyrate, cis-3-héxène-1-ylacétate cis-3-γ-décalactone, éthylcaproate, butylacétate, éthylbenzoate, éthylbutyrate, hexylacétate, méthylcaproate, alcool phényléthylique, citonellol, undecylaldehyde, benzylphénylacétate, alcool cinnamique, eugénol, benzylacétate, alcool linique, cis-jasmone, acétylméthylanthranilate, cis-3-hexène-1-ol, cis-3-hexen-1-ylsalicyclate, méthylbenzoate, méthylsalicylate, géranylacétate, cis-3-hexène-1-ylacétate, lisea cubeba, huile d'orange, alcool phénylpropylique et phényléthylacétate.

25. Dispositif de réfrigération, en particulier réfrigérateur domestique avec un dispositif pour le traitement de l'air selon l'une des revendications 1 à 24.

26. Dispositif de réfrigération selon la revendication 25, **caractérisé en ce que** le dispositif (114) de traitement de l'air est disposé sur un boîtier de dispositif de réfrigération, et le dispositif de traitement de l'air est relié, par un canal (122), à l'espace intérieur (124) du dispositif de réfrigération.

27. Dispositif de réfrigération selon la revendication 26, **caractérisé en ce que** le dispositif (114) de traitement de l'air est disposé à l'intérieur de l'espace intérieur (124) du dispositif de réfrigération.

28. Ordinateur, comprenant un dispositif (114) pour le traitement de l'air selon l'une des revendications 1 à 24, le dispositif (114) de traitement de l'air étant disposé de préférence dans le dispositif d'aération d'ordinateur (134).

29. Ordinateur, selon la revendication 28, **caractérisé en ce que** le transport de l'agent de traitement de l'air s'effectue au moyen du dispositif d'aération d'ordinateur (134).

30. Ordinateur selon l'une des revendications 28 ou 29, **caractérisé en ce que** la chaleur produite par l'ordinateur sert de dispositif d'évaporation.

31. Procédé pour aseptiser et/ou éliminer les odeurs d'un dispositif de réfrigération, en particulier pour aseptiser un réfrigérateur, comprenant l'évaporation d'une composition anti-microbienne et/ou d'une composition masquant les odeurs, dans le dispositif de réfrigération, au moyen d'un dispositif défini, tel que dans les revendications 1 à 24.

32. Procédé pour aseptiser un ordinateur et de l'enceinte dans lequel l'ordinateur est installé, comprenant l'évaporation d'une composition anti-microbienne au moyen d'un dispositif défini aux revendications 1 à 24, disposé dans la zone de l'installation d'aération de l'ordinateur.

33. Procédé selon la revendication 31 ou 32, la composition anti-microbienne contenant une ou plusieurs substances aromatiques GRAS ou bien leurs dérivées.

34. Procédé selon la revendication 32 ou 33, la composition anti-microbienne étant évaporée, en une quantité de 0,01 à 0,5 ml par heure, pour aseptiser un réfrigérateur.

35. Procédé selon l'une des revendications 31 à 34, la concentration de la composition anti-microbienne dans le dispositif de réfrigération, l'ordinateur ou l'enceinte, dans lequel l'ordinateur est installé, étant inférieure ou égale à 500 ppb, de préférence inférieure à 10 ppb.
